(19) Europäisches Patentamt
European Patent Office
Office européen des brevets

(11) EP 2 792 352 A1

(12) **EUROPEAN PATENT APPLICATION**

(43) Date of publication:
**22.10.2014 Bulletin 2014/43**

(21) Application number: **13382140.5**

(22) Date of filing: **16.04.2013**

(51) Int Cl.:
***A61K 31/155*** *(2006.01)* ***A61K 31/415*** *(2006.01)*
***A61K 31/4168*** *(2006.01)* ***A61K 31/5377*** *(2006.01)*
***A61P 25/06*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Laboratorios del. Dr. Esteve, S.A.**
**08041 Barcelona (ES)**

(72) Inventors:
- **Zamanillo-Castanedo, Daniel**
  **E-08041 Barcelona (ES)**
- **Vela Hernández, José Miguel**
  **E-08028 Barcelona (ES)**

(74) Representative: **ABG Patentes, S.L.**
**Avenida de Burgos, 16D**
**Edificio Euromor**
**28036 Madrid (ES)**

(54) **Alpha-2 adrenoreceptor and sigma receptor ligand combinations**

(57) The invention refers to a combination comprising a Sigma ligand of general formula (I) and alpha-2-adrenergic agonist compound, a medicament comprising said active substance combination, and the use of said active substance combination for the manufacture of a medicament, particularly for the prophylaxis and/or treatment of pain.

EP 2 792 352 A1

## Description

### FIELD OF THE INVENTION

The present invention relates to an active substance combination, pharmaceutical compositions containing it and their use in medicine, particularly for the prophylaxis and/or treatment of pain.

### BACKGROUND

The treatment of pain conditions is of great importance in medicine. There is currently a world-wide need for additional pain therapy. The pressing requirement for a specific treatment of pain conditions is documented in the large number of scientific works that have appeared recently in the field of applied analgesics.

PAIN is defined by the International Association for the Study of Pain (IASP) as "an unpleasant sensory and emotional experience associated with actual or potential tissue damage, or described in terms of such damage" (IASP, Classification of chronic pain, 2nd Edition, IASP Press (2002), 210). Although it is a complex process influenced by both physiological and psychological factors and is always subjective, its causes or syndromes can be classified. Pain can be classified based on temporal, aetiological or physiological criteria. When pain is classified by time, it can be acute or chronic. Aetiological classifications of pain are malignant or non-malignant. A third classification is physiological, which includes nociceptive pain (results from detection by specialized transducers in tissues attached to A-delta and C-fibres), that can be divided into somatic and visceral types of pain, and neuropathic pain (results from irritation or damage to the nervous system), that can be divided into peripheral and central neuropathic pain. Pain is a normal physiological reaction of the somatosensory system to noxious stimulation which alerts the individual to actual or potential tissue damage. It serves a protective function of informing us of injury or disease, and usually remits when healing is complete or the condition is cured. However, pain may result from a pathological state characterized by one or more of the following: pain in the absence of a noxious stimulus (spontaneous pain), increased duration of response to brief stimulation (ongoing pain or hyperpathia), reduced pain threshold (allodynia), increased responsiveness to suprathreshold stimulation (hyperalgesia), spread of pain and hyperalgesia to uninjured tissue (referred pain and secondary hyperalgesia), and abnormal sensations (e.g., dysesthesia, paresthesia).

Noradrenaline and alpha-2-adrenoceptors are implicated in the modulation of pain in various behavioural conditions. Noradrenergic neurons and synaptic noradrenergic inputs are present in neuronal circuits critical for pain modulation. Several studies demonstrate that spinal application of noradrenaline or electrical stimulation of cerebral noradrenergic cell nuclei that project to the dorsal horn of the spinal cord elicit robust antinociception (Yaksh, 1985; Eisenach et al., 1998; Buerkle and Yaksh, 1998). There is clinical evidence that alpha-2 adrenergic agonists elicit analgesia against a diversity of painful states, including neuropathic pain (Ongioco et al., 2000; Asano et al., 2000; Hall et al., 2001). Potential sites of action for the analgesic effects of alpha-2 receptor agonists include the brain, spinal cord, dorsal root ganglia and sensory neurons (Sierralta et al., 1996; Asano et al., 2000; Ongioco et al., 2000). The mechanism of action of alpha-2 receptor-mediated analgesia involves a modulation of descending inhibitory pathways implicated in pain control (Nakajima et al., 2012). It is also known that alpha-2 receptor agonists are potent antinociceptive agents having a potency that can be even greater than that reported for morphine (Samso et al., 1996; Gentili et al., 1997; Wilson et al., 2003). However, therapeutic utility of alpha-2 adrenergic agonists is limited by undesirable adverse effects including sedation, dry mouth, hypotension and rebound hypertension (Dias et al., 1999; Puskas et al., 2003).

On another front, the search for new therapeutic agents has been greatly aided in recent years by better understanding of the structure of proteins and other biomolecules associated with target diseases. One important class of these proteins are the sigma (σ) receptors, cell surface receptors of the central nervous system (CNS) which may be related to the dysphoric, hallucinogenic and cardiac stimulant effects of opioids. From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, 1990). It has been reported that the known sigma receptor ligand rimcazole clinically shows effects in the treatment of psychosis (Snyder, 1989). The sigma binding sites have preferential affinity for the dextrorotatory isomers of certain opiate benzomorphans, such as (+)-SKF 10047, (+)-cyclazocine, and (+)-pentazocine and also for some narcoleptics such as haloperidol. Also, sigma receptors are non-opiaceous type of receptors of great interest in pharmacology due to their role in analgesia related processes.

"The sigma receptor/s" as used in this application is/are well known and defined using the following citation: This binding site represents a typical protein different from opioid, NMDA, dopaminergic, and other known neurotransmitter or hormone receptor families (G. Ronsisvalle et al., 2001).

Two subtypes of sigma receptors (Sigma-1 and Sigma-2 receptors) have been identified (Cobos et al., 2008). Confused with opioid receptors for many years due to the cross-reactivity of some ligands, the Sigma-1 receptor is a 24-kDa molecular mass protein of 223 amino acids anchored to the endoplasmic reticulum and plasma membranes

(Cobos et al., 2008; Maurice and Su, 2009).

The Sigma-1 receptor is a non-opiaceous type receptor expressed in numerous adult mammal tissues (e.g. central nervous system, ovary, testicle, placenta, adrenal gland, spleen, liver, kidney, gastrointestinal tract) as well as in embryo development from its earliest stages, and is apparently involved in a large number of physiological functions. Its high affinity for various pharmaceuticals has been described, such as for SKF-10047, (+)-pentazocine, haloperidol and rimcazole, among others, known ligands with analgesic, anxiolytic, antidepressive, antiamnesic, antipsychotic and neuroprotective activity. Sigma-1 receptor is of great interest in pharmacology in view of its possible physiological role in processes related to analgesia, anxiety, addiction, amnesia, depression, schizophrenia, stress, neuroprotection and psychosis (Kaiser et al. 1991; Walker, J.M. et al, 1990 and Bowen W.D., 2000).

Sigma-1 receptor is a unique ligand-regulated molecular chaperone which is activated under stress or pathological conditions and interacts with several neurotransmitter receptors and ion channels to modulate their function. The effects reported preclinically with Sigma-1 receptor ligands are consistent with a role for sigma-1 receptor in central sensitization and pain hypersensitivity and suggest a potential therapeutic use of Sigma-1 receptor antagonists for the management of neuropathic pain as monotherapy (Romero et al., 2012).

Pyrazole derivatives of general formula (I) according to the present invention are described in WO 2006/021462 as compounds having pharmacological activity towards the sigma (δ) receptor useful, inter alia, in the prophylaxis and/or treatment of pain.

Pharmaceutical compositions (WO 2011/064296 A1), salts (WO 2011/064315 A1), polymorphs and solvates (WO 2011/095579 A1), and other solid forms (WO 2012/019984 A1) of said sigma ligands of formula (I) have been also disclosed as well as combinations with other active substances such a with opioids or opiates (WO 2009/130310 A1, WO 2012/016980 A2, WO 2012/072782 A1) or with chemotherapeutic drugs (WO 2011/018487 A1, WO 2011/144721 A1).

As above mentioned, therapeutic utility of alpha-2 adrenergic agonists is limited by undesirable adverse effects including sedation, dry mouth, hypotension and rebound hypertension (Dias et al., 1999; Puskas et al., 2003). Thus, strategies aimed to reduce doses needed for alpha-2 adrenergic receptor agonist analgesia are needed and may improve their therapeutic window and extend their use in clinics.

## BRIEF DESCRIPTION OF THE INVENTION

It is an object of the present invention to provide a medicament suitable for the prophylaxis and/or treatment of pain, which preferably does not show the undesired side effects of the alpha-2 adrenergic agonists used for the prophylaxis and/or treatment of pain, or at least less frequent and/or less pronounced.

The inventors of the present invention have found and demonstrated that the administration of some specific Sigma receptor ligands in conjunction with an alpha-2 adrenergic agonist ligands potentiate synergistically the analgesia.

In particular, the inventors of the present invention have found and demonstrated that the administration of some specific Sigma receptor ligands in conjunction with an alpha-2 adrenergic agonist ligands surprisingly potentiate synergistically the analgesic effect of the alpha-2 adrenergic agonist ligands, indicating that the combination of a Sigma ligand and an alpha-2 adrenergic agonist reduces the doses needed to obtain effective analgesia of the latter.

Furthermore, the inventors of the present invention have found and demonstrated that the administration of some specific Sigma receptor ligands in conjunction with an alpha-2 adrenergic agonist ligands surprisingly potentiate synergistically the analgesic effect of the Sigma ligands.

In particular, the Sigma ligands according to the present invention are Sigma-1 receptor ligands.

More particularly, the Sigma ligands according to the present invention are Sigma-1 antagonist receptor ligands.

Therefore, one aspect of the present invention relates to a combination comprising at least one alpha-2 adrenergic agonist ligand and at least one Sigma ligand of general formula (I), or a pharmaceutically acceptable salt, isomer, prodrug or solvate thereof

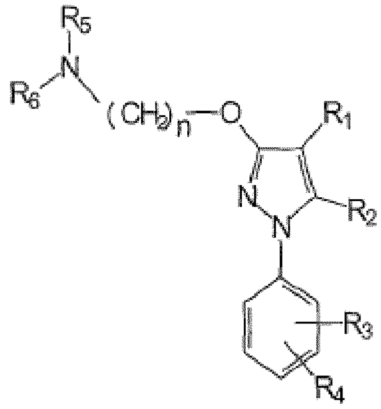

**(I)**

wherein,

$\mathbf{R_1}$ is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, $-COR_8$, $-C(O)OR_8$, $-C(O)NR_8R_9$, $-CH=NR_8$, -CN, $-OR_8$, $-OC(O)R_8$, $-S(O)_t-R_8$, $-NR_8R_9$, $-NR_8C(O)R_9$, $-NO_2$, $-N=CR_8R_9$, and halogen;

$\mathbf{R_2}$ is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, $-COR_8$, $-C(O)OR_8$, $-C(O)NR_8R_9$, $-CH=NR_8$, -CN, $-OR_8$, $-OC(O)R_8$, $-S(O)_t-R_8$, $-NR_8R_9$, $-NR_8C(O)R_9$, $-NO_2$, $-N=CR_8R_9$, and halogen;

$\mathbf{R_3}$ and $\mathbf{R_4}$ are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, $-COR_8$, $-C(O)OR_8$, $-C(O)NR_8R_9$, $-CH=NR_8$, -CN, $-OR_8$, $-OC(O)R_8$, $-S(O)_t-R_8$, $-NR_8R_9$, $-NR_8C(O)R_9$, $-NO_2$, $-N=CR_8R_9$, and halogen, or together with the phenyl they form an optionally substituted fused ring system;

$\mathbf{R_5}$ and $\mathbf{R_6}$ are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, $-COR_8$, $-C(O)OR_8$, $-C(O)NR_8R_9$, $-CH=NR_8$, -CN, $-OR_8$, $-OC(O)R_8$, $-S(O)_t-R_8$, $-NR_8R_9$, $-NR_8C(O)R_9$, $-NO_2$, $-N=CR_8R_9$, and halogen;

or together form, with the nitrogen atom to which they are attached, a substituted or unsubstituted, aromatic or non-aromatic heterocyclyl group;

**n** is selected from 1, 2, 3, 4, 5, 6, 7 and 8;

**t** is 0, 1 or 2;

$\mathbf{R_8}$ and $\mathbf{R_9}$ are each independently selected from hydrogen, substituted or unsubstituted alkyl, substituted or unsubstituted cycloalkyl, substituted or unsubstituted alkenyl, substituted or unsubstituted aryl, substituted or unsubstituted, aromatic or non-aromatic heterocyclyl, substituted or unsubstituted alkoxy, substituted or unsubstituted aryloxy, and halogen.

A further aspect of the invention refers to the Sigma ligand of general formula (I) as defined above, or a pharmaceutically acceptable salt, isomer, prodrug or solvate thereof, for use in potentiating the analgesic effect of an

alpha-2 adrenergic agonist when said alpha-2 adrenergic agonist is used in the prophylaxis and/or treatment of pain.

Another aspect of this invention refers to the use of a Sigma ligand of general formula (I) as defined above, or a pharmaceutically acceptable salt, isomer, prodrug or solvate thereof, for manufacturing a medicament for potentiating the analgesic effect of an alpha-2 adrenergic agonist when said alpha-2 adrenergic agonist is used in the prophylaxis and/or treatment of pain.

Another aspect of this invention refers to the combination comprising at least one Sigma ligand of general formula (I) as defined above, or a pharmaceutically acceptable salt, isomer, prodrug or solvate thereof, and at least one alpha-2 adrenergic agonist for use in the prophylaxis and/or treatment of pain.

Another aspect of this invention refers to the use of the combination comprising at least one Sigma ligand of general formula (I) as defined above, or a pharmaceutically acceptable salt, isomer, prodrug or solvate thereof, and at least one alpha-2 adrenergic agonist for manufacturing a medicament for the prophylaxis and/or treatment of pain.

Another aspect of this invention refers to the combination, for simultaneous, separated or sequential administration, comprising at least one Sigma ligand of general formula (I) as defined above, or a pharmaceutically acceptable salt, isomer, prodrug or solvate thereof, and at least one alpha-2 adrenergic agonist for use in the prophylaxis and/or treatment of pain.

Another aspect of this invention refers to the use of the combination, for simultaneous, separated or sequential administration, comprising at least one Sigma ligand of general formula (I) as defined above, or a pharmaceutically acceptable salt, isomer, prodrug or solvate thereof, and at least one alpha-2 adrenergic agonist for manufacturing a medicament for the prophylaxis and/or treatment of pain.

Another aspect of the invention is a method of treatment and/or prophylaxis of a patient suffering from pain, or likely to suffer pain, the method comprising administering to the patient in need of such a treatment or prophylaxis a therapeutically effective amount of a combination comprising at least one Sigma ligand of general formula (I) as defined above, or a pharmaceutically acceptable salt, isomer, prodrug or solvate thereof, and at least one alpha-2 adrenergic agonist.

These aspects and preferred embodiments thereof are additionally also defined hereinafter in the detailed description, as well as in the claims.

## BRIEF DESCRIPTION OF THE FIGURES

**Figure 1:** Dose-response effect of acute administration of Sigma ligand compound 63·HCl (5, 10, and 40 mg/kg, ip) in the tail-flick test in male CD-1 mice, alone and in combination with clonidine (0.125 mg/kg, sc). Compounds were administered 30 min before the test. 5-13 animals per group were used. Data are presented as the mean±SEM of the tail withdrawal latency (s). *p<0.05, ***p<0.001 vs. vehicle treated group (saline + saline); #p<0.05 vs. clonidine treated group (saline + clonidine 0.125 mg/kg) (Newman-Keuls Multiple Comparison Test post-one way ANOVA).

**Figure 2**: (A) Dose-response effect of acute administration of clonidine (0.125, 0.25, 0.5 and 1 mg/kg, sc) in the tail-flick test in male CD-1 mice, alone and in combination with Sigma ligand compound 63·HCl (40 mg/kg, ip). Compounds were administered 30 min before the test. 9-10 animals per group were used. Data are presented as the % MPE±SEM. (B) The effective dose 50 (ED50) values for clonidine and clonidine combined with Compound 63·HCl. *** p<0.001 (Unpaired t-test).

**Figure 3:** Effect of acute administration of guanfacine (Gua, 1.25, mg/kg, sc) in the tail-flick test in male CD-1 mice, alone and in combination with Sigma ligand compound 63·HCl (40 mg/kg, ip). Compounds were administered 30 min before the test. 5-13 animals per group were used. Data are presented as the mean±SEM of the tail withdrawal latency. * p<0.05 vs saline + HPMC; # p<0.05 vs Gua 1.25 + HPMC.

**Figura 4:** Effects of acute administration of Sigma ligand Compound 63·HCl (40 mg/kg, ip) in the hot-plate test in male CD-1 mice, alone and in combination with clonidine 0.25 mg/kg, sc. Compound 63·HCl was administered 30 min before the test. 9-10 animals per group were used. Data are presented as the mean±SEM of the latency to hind paw licking (HPL). ***p<0.001 vs. vehicle treated group (control); ###p<0.001 vs. clonidine alone treated group (Newman-Keuls Multiple Comparison Test post-one way ANOVA).

**Figure 5:** Dose-response effect of acute administration of clonidine (0.125, 0.25, 0.5, and 1 mg/kg, sc) in the hot test in male CD-1 mice, alone and in combination with Sigma ligand compound 63·HCl (40 mg/kg, ip.). Compounds were administered 30 min before the test. 8-12 animals per group were used. Data are presented as (A) the mean±SEM of the latency to hind paw licking (HPL) or as (B) the %MPE±SEM. ***p<0.001 vs. clonidine treated

group (Newman-Keuls Multiple Comparison Test post-one way ANOVA).

**Figure 6:** Effect of acute administration of guanfacine (Gua, 5 mg/kg, s.c.) and dexmedetomidine (Dex, 0.01 mg/kg, s.c.) in the hot-plate test in male CD-1 mice, alone and in combination with Sigma ligand compound 63·HCl (40 mg/kg, ip.). Compounds were administered 30 min before the test. 6-10 animals per group were used. Data are presented as the mean±SEM of the latency to hind paw licking (HPL). * $p<0.05$ vs control (saline + HPMC).

## DETAILED DESCRIPTION OF THE INVENTION

In the context of the present invention, the following terms have the meaning detailed below.

"Alkyl" refers to a straight or branched hydrocarbon chain radical containing no unsaturation, and which is attached to the rest of the molecule by a single bond. Typical alkyl groups have from 1 to about 12, 1 to about 8, or 1 to about 6 carbon atoms, e. g., methyl, ethyl, n-propyl, i-propyl, n-butyl, t-butyl, n-pentyl, etc. If substituted by aryl, it corresponds to an "arylalkyl" radical, such as benzyl or phenethyl. If substituted by heterocyclyl, it corresponds to a "heterocyclylalkyl" radical.

"Alkenyl" refers to a straight or branched hydrocarbon chain radical containing at least two carbon atoms and at least one unsaturation, and which is attached to the rest of the molecule by a single bond. Typical alkenyl radicals have from 2 to about 12, 2 to about 8 or 2 to about 6 carbon atoms. In a particular embodiment, the alkenyl group is vinyl, 1-methyl-ethenyl, 1-propenyl, 2-propenyl, or butenyl.

"Alkynyl" refers to a straight or branched hydrocarbon chain radical containing at least two carbon atoms and at least one carbon-carbon triple bond, and which is attached to the rest of the molecule by a single bond. Typical alkynyl radicals have from 2 to about 12, 2 to about 8 or 2 to about 6 carbon atoms. In a particular embodiment, the alkynyl group is ethynyl, propynyl (e.g. 1-propynyl, 2-propynyl), or butynyl (e.g. 1-butynyl, 2-butynyl, 3-butynyl).

"Cycloalkyl" refers to an alicyclic hydrocarbon. Typical cycloalkyl radicals contain from 1 to 3 separated and/or fused rings and from 3 to about 18 carbon atoms, preferably from 3 to 10 carbon atoms, such as cyclopropyl, cyclohexyl or adamantyl. In a particular embodiment, the cycloalkyl radical contains from 3 to about 6 carbon atoms.

"Aryl" refers to single and multiple ring radicals, including multiple ring radicals that contain separate and/or fused aryl groups. Typical aryl groups contain from 1 to 3 separated or fused rings and from 6 to about 18 carbon ring atoms, such as phenyl, naphthyl (e.g. 2-naphthyl), indenyl, fenanthryl or anthracyl radical.

"Heterocyclyl" refers to a stable, typically 3- to 18-membered, ring radical which consists of carbon atoms and from one to five heteroatoms selected from the group consisting of nitrogen, oxygen, and sulfur, preferably a 4- to 8-membered ring with one or more heteroatoms, more preferably a 5- or 6-membered ring with one or more heteroatoms. It may be aromatic or not aromatic. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized ; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran, coumarine, morpholine; pyrrole, pyrazole, oxazole, isoxazole, triazole, imidazole, etc.

"Alkoxy" refers to a radical of the formula $-OR_a$ where $R_a$ is an alkyl radical as defined above having one or more (e.g., 1, 2, 3 or 4) oxygen linkages and typically from 1 to about 12, 1 to about 8 or 1 to about 6 carbon atoms, e. g., methoxy, ethoxy, propoxy, etc.

"Aryloxy" refers to a radical of formula -O-aryl, where aryl is as previously defined. Some examples of aryloxy compounds are -O-phenyl, -O-p-tolyl, -O-m-tolyl, - O-o-tolyl or -O-naphthyl.

"Amino" refers to a radical of the formula $-NH_2$, $-NHR_a$ or $-NR_aR_b$, optionally quaternized. In an embodiment of the invention each of $R_a$ and $R_b$ is independently selected from hydrogen and an alkyl radical as defined above e.g., methylamino, ethylamino, dimethylamino, diethylamino, propylamino, etc..

"Halogen", "halo" or "hal" refers to bromo, chloro, iodo or fluoro.

"Fused ring system" refers to a polycyclic ring system that contains fused rings. Typically, the fused ring system contains 2 or 3 rings and/or up to 18 ring atoms. As defined above, cycloalkyl radicals, aryl radicals and heterocyclyl radicals may form fused ring systems. Thus, fused ring system may be aromatic, partially aromatic or not aromatic and may contain heteroatoms. A spiro ring system is not a fused-polycyclic by this definition, but fused polycyclic ring systems of the invention may themselves have spiro rings attached thereto via a single ring atom of the system. Examples of fused ring systems are, but are not limited to, adamantyl, naphthyl (e.g. 2-naphthyl), indenyl, fenanthryl, anthracyl, pyrenyl, benzimidazole, benzothiazole, etc..

Unless otherwise stated specifically in the specification, all the groups may be optionally substituted, if applicable. References herein to substituted groups in the compounds of the present invention refer to the specified moiety that may be substituted at one or more (e.g., 1, 2, 3 or 4) available positions by one or more suitable groups, e. g., halogen such as fluoro, chloro, bromo and iodo ; cyano; hydroxyl ; nitro ; azido ; acyl, such as alkanoyl, e.g. a $C_{1-6}$ alkanoyl group,

and the like; carboxamido; alkyl groups including those groups having 1 to about 12 carbon atoms or from 1 to about 6 carbon atoms and more preferably 1-3 carbon atoms; alkenyl and alkynyl groups including groups having one or more (e.g., 1, 2, 3 or 4) unsaturated linkages and from 2 to about 12 carbon or from 2 to about 6 carbon atoms; alkoxy groups having one or more (e.g., 1, 2, 3 or 4) oxygen linkages and from 1 to about 12 carbon atoms or 1 to about 6 carbon atoms; aryloxy such as phenoxy; alkylthio groups including those moieties having one or more (e.g., 1, 2, 3 or 4) thioether linkages and from 1 to about 12 carbon atoms or from 1 to about 6 carbon atoms; alkylsulfinyl groups including those moieties having one or more (e.g., 1, 2, 3 or 4) sulfinyl linkages and from 1 to about 12 carbon atoms or from 1 to about 6 carbon atoms ; alkylsulfonyl groups including those moieties having one or more (e.g., 1, 2, 3 or 4) sulfonyl linkages and from 1 to about 12 carbon atoms or from 1 to about 6 carbon atoms; aminoalkyl groups such as groups having one or more (e.g., 1, 2, 3 or 4) N atoms and from 1 to about 12 carbon atoms or from 1 to about 6 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

The term "salt" must be understood as any form of a compound used in accordance with this invention in which said compound is in ionic form or is charged and coupled to a counter-ion (a cation or anion) or is in solution. This definition also includes quaternary ammonium salts and complexes of the molecule with other molecules and ions, particularly, complexes formed via ionic interactions. The definition includes in particular physiologically acceptable salts; this term must be understood as equivalent to "pharmacologically acceptable salts" or "pharmaceutically acceptable salts".

The term "pharmaceutically acceptable salts" in the context of this invention means any salt that is tolerated physiologically (normally meaning that it is not toxic, particularly, as a result of the counter-ion) when used in an appropriate manner for a treatment, applied or used, particularly, in humans and/or mammals. These physiologically acceptable salts may be formed with cations or bases and, in the context of this invention, are understood to be salts formed by at least one compound used in accordance with the invention -normally an acid (deprotonated)- such as an anion and at least one physiologically tolerated cation, preferably inorganic, particularly when used on humans and/or mammals. Salts with alkali and alkali earth metals are preferred particularly, as well as those formed with ammonium cations ($NH_4^+$). Preferred salts are those formed with (mono) or (di)sodium, (mono) or (di)potassium, magnesium or calcium. These physiologically acceptable salts may also be formed with anions or acids and, in the context of this invention, are understood as being salts formed by at least one compound used in accordance with the invention - normally protonated, for example in nitrogen - such as a cation and at least one physiologically tolerated anion, particularly when used on humans and/or mammals. This definition specifically includes in the context of this invention a salt formed by a physiologically tolerated acid, i.e. salts of a specific active compound with physiologically tolerated organic or inorganic acids - particularly when used on humans and/or mammals. Examples of this type of salts are those formed with: hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid.

The term "solvate" in accordance with this invention should be understood as meaning any form of a compound in accordance with the invention in which said compound is bonded by a non-covalent bond to another molecule (normally a polar solvent), including especially hydrates and alcoholates, like for example, methanolate. A preferred solvate is the hydrate.

Any compound that is a prodrug of the Sigma ligand of formula (I) is also within the scope of the invention. The term "prodrug" is used in its broadest sense and encompasses those derivatives that are converted in vivo to the compounds of the invention. Examples of prodrugs include, but are not limited to, derivatives of the compounds of formula (I) that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger "Medicinal Chemistry and Drug Discovery 6th ed. (Donald J. Abraham ed., 2001, Wiley) and "Design and Applications of Prodrugs" (H. Bundgaard ed., 1985, Harwood Academic Publishers).

Any compound referred to herein is intended to represent such specific compound as well as certain variations or forms. In particular, compounds referred to herein may have asymmetric centres and therefore exist in different enantiomeric or diastereomeric forms. Thus, any given compound referred to herein is intended to represent any one of a racemate, one or more enantiomeric forms, one or more diastereomeric forms, and mixtures thereof. Likewise, stereoisomerism or geometric isomerism about the double bond is also possible, therefore in some cases the molecule could exist as (E)-isomer or (Z)-isomer (trans and cis isomers). If the molecule contains several double bonds, each double bond will have its own stereoisomerism, that could be the same as, or different to, the stereoisomerism of the other double bonds of the molecule. Furthermore, compounds referred to herein may exist as atropisomers. All the stereoisomers including enantiomers, diastereoisomers, geometric isomers and atropisomers of the compounds referred

to herein, and mixtures thereof, are considered within the scope of the present invention.

Furthermore, any compound referred to herein may exist as tautomers. Specifically, the term tautomer refers to one of two or more structural isomers of a compound that exist in equilibrium and are readily converted from one isomeric form to another. Common tautomeric pairs are enamine-imine, amide-imidic acid, keto-enol, lactam-lactim, etc.

Unless otherwise stated, the compounds of the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by $^{13}C$- or $^{14}C$-enriched carbon, or the replacement of at least one nitrogen by $^{15}N$-enriched nitrogen are within the scope of this invention.

The compounds of the invention or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrug.

As used herein, the terms "treat", "treating" and "treatment" include the eradication, removal, reversion, alleviation, modification, or control of pain after its onset.

As used herein, the terms "prevention", "preventing", "preventive" "prevent" and "prophylaxis" refer to the capacity of a therapeutic to avoid, minimize or difficult the onset or development of a disease or condition before its onset, in this case pain.

Therefore, by "treating" or "treatment" and/or "preventing" or "prevention", as a whole, is meant at least a suppression or an amelioration of the symptoms associated with the condition afflicting the subject, where suppression and amelioration are used in a broad sense to refer to at least a reduction in the magnitude of a parameter, e.g., symptom associated with the condition being treated, such as pain. As such, the method of the present invention also includes situations where the condition is completely inhibited, e.g., prevented from happening, or stopped, e.g., terminated, such that the subject no longer experiences the condition. As such, the present method includes both preventing and managing pain, particularly, peripheral neuropathic pain, allodynia, causalgia, hyperalgesia, hyperesthesia, hyperpathia, neuralgia, neuritis or neuropathy.

As used herein, the term "potentiating the analgesic effect of an alpha-2 adrenergic agonist" refers to the increase in the effectiveness of the analgesic effect of said alpha-2 adrenergic agonist produced by sigma ligands of formula (I). In an embodiment of the invention, said potentiating effect induces an increase in the analgesic effect of alpha-2 adrenergic agonist by a factor of 1.2, 1.5, 2, 3, 4 or more when compared with the alpha-2 adrenergic agonist when administered in isolation. The measurement can be done following any known method in the art.

As used herein, the term "potentiating the analgesic effect of Sigma ligand of formula (I)" refers to the increase in the effectiveness of the analgesic effect of said Sigma ligand of formula (I) produced by alpha-2 adrenergic agonists. In an embodiment of the invention said potentiating effect induces an increase in the analgesic effect of Sigma ligand of formula (I) by a factor of 1.2, 1.5, 2, 3, 4 or more when compared with the Sigma ligand of formula (I) when administered in isolation. The measurement can be done following any known method in the art.

An "agonist" is defined as a compound that binds to a receptor and has an intrinsic effect, and thus, increases the basal activity of a receptor when it contacts the receptor.

Alpha-2-adrenergic agonists include chemical entities, such as compounds, ions, complexes and the like, which are effective to act on or bind to alpha-2-adrenergic receptors and provide a therapeutic effect. It is a well known class of drugs, that are used for example as anesthetics. Alpha-2-adrenergic agonists means the agonists themselves and any and all precursors thereof, metabolites thereof and combinations thereof.

In one embodiment, the alpha-2-adrenergic agonist is selected from the group consisting of imino-imidazolines, imidazolines, imidazoles, azepines, thiazines, oxazolines, guanidines, catecholamines, derivatives thereof and mixtures thereof.

Without limiting the invention to the specific groups and compounds listed, the following is a list of representative alpha-2 adrenergic agonists useful in this invention: imino-imidazolines, including clonidine, apraclonidine and tizanidine; imidazolines, including naphazoline, xymetazoline, tetrahydrozoline, and tramazoline; imidazoles, including fadolmidine detomidine, medetomidine, and dexmedetomidine; azepines, including B-HT 920 (6-allyl-2-amino-5,6,7,8 tetrahydro-4H-thiazolo[4,5-d]-azepine and B-HT 933; thiazines, including xylazine; oxazolines, including rilmenidine; guanidines, including guanabenz, guanfacine and guanethidine; catecholamines, including methyldopa; and the like and derivatives thereof.

Examples of alpha-2 adrenergic agonists useful in the present invention according to a particular embodiment include, but are not limited to: clonidine, apraclonidine, tizanidine, naphazoline, xymetazoline, tetrahydrozoline, tramazoline, fadolmidine, detomidine, medetomidine, dexmedetomidine, B-HT 920 (6-allyl-2-amino-5,6,7,8 tetrahydro-4H-thiazolo[4,5-d]-azepine) and B-HT 933, xylazine, rilmenidine, guanabenz, guanoxabenz, guanfacine, guanethidine, and

methyldopa.

In a preferred embodiment, the alpha-2 adrenergic agonist ligand is clonidine, tizanidine, dexmedetomidine or guanfacine.

As above mentioned, the Sigma ligands of general formula (I) surprisingly potentiate the analgesic effect of alpha-2 adrenergic agonists, thus reducing the doses needed to obtain effective analgesia of the latter.

In a preferred embodiment, $R_1$ in the compounds of general formula (I) is selected from H, -$COR_8$, and substituted or unsubstituted alkyl. More preferably, $R_1$ is selected from H, methyl and acetyl. A more preferred embodiment is when $R_1$ is H.

In another preferred embodiment, $R_2$ in the compounds of formula (I) represents H or alkyl, more preferably methyl.

In a particular embodiment of the invention, $R_3$ and $R_4$ in the compounds of formula (I) are situated in the meta and para positions of the phenyl group, and preferably, they are selected independently from halogen and substituted or unsubstituted alkyl.

In an especially preferred embodiment of the invention, in the compounds of formula (I) both $R_3$ and $R_4$ together with the phenyl group form an optionally substituted fused ring system. More preferably, said fused ring system is selected from a substituted or unsubstituted fused aryl group and a substituted or unsubstituted aromatic or partially aromatic fused heterocyclyl group. Said fused ring system preferably contains two rings and/or from 9 to about 18 ring atoms, more preferably 9 or 10 ring atoms. Even more preferably, the fused ring system is naphthyl, especially a 2-naphthyl ring system, substituted or unsubstituted.

Also in the compounds of formula (I), embodiments where n is selected from 2, 3 or 4 are preferred in the context of the present invention, more preferably n is 2.

Finally, in another embodiment it is preferred in the compounds of formula (I) that $R_5$ and $R_6$ are, each independently, $C_{1-6}$alkyl, or together with the nitrogen atom to which they are attached form a substituted or unsubstituted heterocyclyl group a, in particular a group chosen among morpholinyl, piperidinyl, and pyrrolidinyl group. More preferably, $R_5$ and $R_6$ together form a morpholine-4-yl group.

In preferred variants of the invention, the Sigma ligand of general formula (I) is selected from:

[1] 4-{2-(1-(3,4-dichlorophenyl)-5-methyl-1H pyrazol-3-yloxy)ethyl} morpholine,

[2] 2-[1-(3,4-Dichlorophenyl)-5-methyl-1H-pyrazol-3-yloxy]-N,N-diethylethanamine,

[3] 1-(3,4-Dichlorophenyl)-5-methyl-3-[2-(pyrrolidin-1-yl)ethoxy]-1H-pyrazole,

[4] 1-(3,4-Dichlorophenyl)-5-methyl-3-[3-(pyrrolidin-1-yl)propoxy]-1H-pyrazole,

[5] 1-{2-[1-(3,4-Dichlorophenyl)-5-methyl-1H-pyrazol-3-yloxy]ethyl}piperidine,

[6] 1-{2-[1-(3,4-dichlorophenyl)-5-methyl-1H-pyrazol-3-yloxy]ethyl}-1H-imidazole,

[7] 3-{1-[2-(1-(3,4-Dichlorophenyl)-5-methyl-1H-pyrazol-3-yloxy)ethyl]piperidin-4-yl}-3H-imidazo[4,5-b]pyridine,

[8]1-{2-[1-(3,4-Dichlorophenyl)-5-methyl-1H-pyrazol-3-yloxy]ethyl}-4-methylpiperazine,

[9] Ethyl 4-{2-[1-(3,4-dichlorophenyl)-5-methyl-1H-pyrazol-3-yloxy]ethyl} piperazine carboxylate,

[10] 1-(4-(2-(1-(3,4-dichlorophenyl)-5-methyl-1H-pyrazol-3-yloxy)ethyl)piperazin-1-yl)ethanone,

[11] 4-{2-[1-(4-Methoxyphenyl)-5-methyl-1H-pyrazol-3-yloxy]ethyl}morpholine,

[12] 1-(4-Methoxyphenyl)-5-methyl-3-[2-(pyrrolidin-1-yl)ethoxy]-1H-pyrazole,

[13] 1-(4-Methoxyphenyl)-5-methyl-3-[3-(pyrrolidin-1-yl)propoxy]-1H-pyrazole,

[14] 1-[2-(1-(4-Methoxyphenyl)-5-methyl-1H-pyrazol-3-yloxy)ethyl]piperidine,

[15]1-{2-[1-(4-Methoxyphenyl)-5-methyl-1H-pyrazol-3-yloxy]ethyl}-1H-imidazole,

[16] 4-{2-[1-(3,4-Dichlorophenyl)-5-phenyl-1H-pyrazol-3-yloxy]ethyl} morpholine,

[17] 1-(3,4-Dichlorophenyl)-5-phenyl-3-[2-(pyrrolidin-1-yl)ethoxy]-1H-pyrazole,

[18] 1-(3,4-Dichlorophenyl)-5-phenyl-3-[3-(pyrrolidin-1-yl)propoxy]-1H-pyrazole,

[19] 1-{2-[1-(3,4-Dichlorophenyl)-5-phenyl-1H-pyrazol-3-yloxy]ethyl}piperidine,

[20] 1-{2-[1-(3,4-Dichlorophenyl)-5-phenyl-1H-pyrazol-3-yloxy]ethyl}-1H-imidazole,

[21]2-{2-[1-(3,4-dichlorophenyl)-5-phenyl-1H-pyrazol-3-yloxy]ethyl}-1,2,3,4-tetrahydroisoquinoline,

[22] 4-{4-[1-(3,4-Dichlorophenyl)-5-methyl-1H-pyrazol-3-yloxy]butyl} morpholine,

[23] 1-(3,4-Dichlorophenyl)-5-methyl-3-[4-(pyrrolidin-1-yl)butoxy]-1H-pyrazole,

[24] 1-{4-[1-(3,4-Dichlorophenyl)-5-methyl-1H-pyrazol-3-yloxy]butyl}piperidine,

[25]1-{4-[1-(3,4-Dichlorophenyl)-5-methyl-1H-pyrazol-3-yloxy]butyl}-4-methylpiperazine,

[26] 1-{4-[1-(3,4-Dichlorophenyl)-5-methyl-1H-pyrazol-3-yloxy]butyl}-1H-imidazole,

[27] 4-[1-(3,4-Dichlorophenyl)-5-methyl-1H-pyrazol-3-yloxy]-N,N-diethylbutan-1-amine,

[28]1-{4-[1-(3,4-dichlorophenyl)-5-methyl-1H-pyrazol-3-yloxy]butyl}-4-phenylpiperidine,

[29] 1-{4-[1-(3,4-dichlorophenyl)-5-methyl-1H-pyrazol-3-yloxy]butyl}-6,7-dihydro-1H-indol-4(5H)-one,

[30] 2-{4-[1-(3,4-dichlorophenyl)-5-methyl-1H-pyrazol-3-yloxy]butyl}-1,2,3,4-tetrahydroisoquinoline,

[31] 4-{2-[1-(3,4-dichlorophenyl)-5-isopropyl-1H-pyrazol-3-yloxy]ethyl} morpholine,

[32]2-[1-(3,4-Dichlorophenyl)-5-isopropyl-1H-pyrazol-3-yloxy]-N,N-diethylethanamine,

[33] 1-(3,4-Dichlorophenyl)-5-isopropyl-3-[2-(pyrrolidin-1-yl)ethoxy]-1H-pyrazole,

[34] 1-(3,4-Dichlorophenyl)-5-isopropyl-3-[3-(pyrrolidin-1-yl)propoxy]-1H-pyrazole,

[35] 1-{2-[1-(3,4-Dichlorophenyl)-5-isopropyl-1H-pyrazol-3-yloxy]ethyl} piperidine,

[36] 2-{2-[1-(3,4-dichlorophenyl)-5-isopropyl-1H-pyrazol-3-yloxy]ethyl}-1,2,3,4-tetrahydroisoquinoline,

[37] 4-{2-[1-(3,4-dichlorophenyl)-1H-pyrazol-3-yloxy]ethyl}morpholine,

[38] 2-[1-(3,4-dichlorophenyl)-1H-pyrazol-3-yloxy] N,N-diethylethanamine,

[39] 1-(3,4-dichlorophenyl)-3-[2-(pyrrolidin-1-yl)ethoxy]-1H-pyrazole,

[40] 1-{2-[1-(3,4-dichlorophenyl)-1H-pyrazol-3-yloxy]ethyl}piperidine,

[41] 1-(3,4-dichlorophenyl)-3-[3-(pyrrolidin-1-yl)propoxy]-1H-pyrazole,

[42]1-{2-[1-(3,4-Dichlorophenyl)-5-methyl-1H-pyrazol-3-yloxy]ethyl}piperazine,

[43] 1-{2-[1-(3,4-Dichlorophenyl)-5-methyl-1H-pyrazol-3-yloxy]ethyl}pyrrolidin-3-amine,

[44]4-{2-[1-(3,4-Dichlorophenyl)-4,5-dimethyl-1H-pyrazol-3-yloxy]ethyl} morpholine,

[46]2-[1-(3,4-Dichlorophenyl)-4,5-dimethyl-1H-pyrazol-3-yloxy]-N,N-diethylethanamine,

[47] 1-(3,4-Dichlorophenyl)-4,5-dimethyl-3-[2-(pyrrolidin-1-yl)ethoxy]-1H-pyrazole,

[48] 1-(3,4-Dichlorophenyl)-4,5-dimethyl-3-[3-(pyrrolidin-1-yl)propoxy]-1H-pyrazole,

[49] 1-{2-[1-(3,4-Dichlorophenyl)-4,5-dimethyl-1H-pyrazol-3-yloxy]ethyl} piperidine,

[50] 4-{4-[1-(3,4-dichlorophenyl)-1H-pyrazol-3-yloxy]butyl}morpholine,

[51]((2S,6R)-4-{4-[1-(3,4-dichlorophenyl)-1H-pyrazol-3-yloxy]butyl}-2,6-dimethylmorpholine,

[52] 1-{4-[1-(3,4-Dichlorophenyl)-1H-pyrazol-3-yloxy]butyl}piperidine,

[53] 1-(3,4-Dichlorophenyl)-3-[4-(pyrrolidin-1-yl)butoxy]-1H-pyrazole,

[55] 4-[1-(3,4-dichlorophenyl)-1H-pyrazol-3-yloxy]-N,N-diethylbutan-1-amine,

[56] N-benzyl-4-[1-(3,4-dichlorophenyl)-1H-pyrazol-3-yloxy]-N-methylbutan-1-amine,

[57]4-[1-(3,4-dichlorophenyl)-1H-pyrazol-3-yloxy]-N-(2-methoxyethyl)-N-methylbutan-1-amine,

[58] 4-{4-[1-(3,4-dichlorophenyl)-1H-pyrazol-3-yloxy]butyl}thiomorpholine,

[59]1-[1-(3,4-Dichlorophenyl)-5-methyl-3-(2-morpholinoethoxy)-1H-pyrazol-4-yl]ethanone,

[60]1-{1-(3,4-dichlorophenyl)-5-methyl-3-[2-(pyrrolidin-1-yl)ethoxy]-1H-pyrazol-4-yl}ethanone,

[61] 1-{1-(3,4-dichlorophenyl)-5-methyl-3-[2-(piperidin-1-yl)ethoxy]-1H-pyrazol-4-yl}ethanone,

[62] 1-{1-(3,4-dichlorophenyl)-3-[2-(diethylamino)ethoxy]-5-methyl-1H-pyrazol-4-yl}ethanone,

[63] 4-{2-[5-Methyl-1-(naphthalen-2-yl)-1H-pyrazol-3-yloxy]ethyl}morpholine,

[64] N,N-Diethyl-2-[5-methyl-1-(naphthalen-2-yl)-1H-pyrazol-3-yloxy] ethanamine,

[65] 1-{2-[5-Methyl-1-(naphthalen-2-yl)-1H-pyrazol-3-yloxy]ethyl}piperidine, and

[66] 5-Methyl-1-(naphthalen-2-yl)-3-[2-(pyrrolidin-1-yl)ethoxy]-1H-pyrazole,

or their pharmaceutically acceptable salts, solvates or prodrugs.

In a preferred variant of the invention, the Sigma ligand of general formula (I) is 4-{2-[5-methyl-1-(naphthalen-2-yl)-1H-pyrazol-3-yloxy]ethyl} morpholine or a salt thereof.

Preferably, the compound of general formula (I) used is 4-{2-[5-methyl-1-(naphthalen-2-yl)-1H-pyrazol-3-yloxy]ethyl}morpholine hydrochloride.

These particular compounds are designated in the examples of the present invention as compound 63 and compound 63·HCl.

The compounds of formula (I) and their salts or solvates can be prepared as disclosed in the previous application WO2006/021462.

The present invention refers also to the use of medicaments or pharmaceutical compositions comprising at least one Sigma ligand of general formula (I) as defined above, or a pharmaceutically acceptable salt, isomer, prodrug or solvate thereof, and at least one alpha-2 adrenergic agonist combined jointly or separately, together with at least a pharmaceutically acceptable excipient.

The term "excipient" refers to components of a drug compound other than the active ingredient (definition obtained from the European Medicines Agency- EMA). They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong in vivo drug actions, decrease drug metabolism, and reduce drug toxicity. Carriers are also used in designs to increase the effectiveness of drug delivery to the target sites of pharmacological actions (U.S. National Library of Medicine. National Institutes of

Health). Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker, © 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin. The selection of these excipients and the amounts to be used will depend on the form of application of the pharmaceutical composition.

The pharmaceutical composition used according to the present invention can be adapted to any form of administration, be it orally or parenterally, for example pulmonarily, nasally, rectally and/or intravenously. Therefore, the formulation according to the present invention may be adapted for topical or systemic application, particularly for dermal, subcutaneous, intramuscular, intra-articular, intraperitoneal, pulmonary, buccal, sublingual, nasal, percutaneous, vaginal, oral or parenteral application. The preferred form of rectal application is by means of suppositories.

Suitable preparations for oral applications are tablets, pills, chewing gums, capsules, granules, drops or syrups. Suitable preparations for parenteral applications are solutions, suspensions, reconstitutable dry preparations or sprays.

The combination of the invention may be formulated as deposits in dissolved form or in patches, for percutaneous application. Skin applications include ointments, gels, creams, lotions, suspensions or emulsions.

The combination of the invention may be formulated for its simultaneous, separate or sequential administration, with at least a pharmaceutically acceptable excipient. This has the implication that the combination of the Sigma ligand of general formula (I) and the alpha-2-adrenergic agonist may be administered:

a) As a combination that is being part of the same medicament formulation, both being then administered always simultaneously.

b) As a combination of two units, each with one of them giving rise to the possibility of simultaneous, sequential or separate administration. In a particular embodiment, the Sigma ligand of general formula (I) is independently administered from the alpha-2-adrenergic agonist (i.e in two units) but at the same time. In another particular embodiment, the sigma ligand of general formula (I) is administered first, and then the alpha-2-adrenergic agonist is separately or sequentially administered. In yet another particular embodiment, the alpha-2-adrenergic agonist is administered first, and then the Sigma ligand of general formula (I) is administered, separately or sequentially, as defined.

In a particular embodiment of the present invention, the pain is selected from peripheral neuropathic pain, allodynia, causalgia, hyperalgesia, hyperesthesia, hyperpathia, neuralgia, neuritis or neuropathy. More preferably, the pain is hyperalgesia or mechanical allodynia.

"Neuropathic pain" is defined by the IASP as "pain initiated or caused by a primary lesion or dysfunction in the nervous system" (IASP, Classification of chronic pain, 2nd Edition, IASP Press (2002), 210). For the purpose of this invention this term is to be treated as synonymous to "Neurogenic Pain" which is defined by the IASP as "pain initiated or caused by a primary lesion, dysfunction or transitory perturbation in the peripheral or central nervous system".

According to the IASP "peripheral neuropathic pain" is defined as "a pain initiated or caused by a primary lesion or dysfunction in the peripheral nervous system" and "peripheral neurogenic pain" is defined as "a pain initiated or caused by a primary lesion, dysfunction or transitory perturbation in the peripheral nervous system" (IASP, Classification of chronic pain, 2nd Edition, IASP Press (2002), 213).

According to the IASP "allodynia" is defined as "a pain due to a stimulus which does not normally provoke pain" (IASP, Classification of chronic pain, 2nd Edition, IASP Press (2002), 210).

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

According to the IASP "hyperalgesia" is defined as "an increased response to a stimulus which is normally painful" (IASP, Classification of chronic pain, 2nd Edition, IASP Press (2002), 211).

According to the IASP "hyperesthesia" is defined as "increased sensitivity to stimulation, excluding the senses" (IASP, Classification of chronic pain, 2nd Edition, IASP Press (2002), 211).

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition, IASP Press (2002), 212).

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold Increased response | Stimulus and response rate may be the same or different |

According to the IASP "neuralgia" is defined as "pain in the distribution of a nerve or nerves" (IASP, Classification of chronic pain, 2nd Edition, IASP Press (2002), 212).

According to the IASP "neuritis" is defined as "inflammation of a nerve or nerves" (IASP, Classification of chronic pain, 2nd Edition, IASP Press (2002), 212).

According to the IASP "neuropathy/neuritis" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

Another aspect of the invention is a method of treatment and/or prophylaxis of a patient suffering from pain, or likely to suffer pain, the method comprising administering to the patient in need of such a treatment or prophylaxis a therapeutically effective amount of a combination comprising at least one Sigma ligand of general formula (I) as defined above, or a pharmaceutically acceptable salt, isomer, prodrug or solvate thereof, and at least one alpha-2 adrenergic agonist.

By an "effective" amount or a "therapeutically effective amount" of a drug or pharmacologically active agent is meant a nontoxic but sufficient amount of the drug or agent to provide the desired effect. In the combination therapy of the present invention, an "effective amount" of one component of the combination (i.e. Sigma ligand of general formula (I) or alpha-2 adrenergic agonist) is the amount of that compound that is effective to provide the desired effect when used in combination with the other component of the combination (i.e. alpha-2 adrenergic agonist or Sigma ligand of general formula (I)). The amount that is "effective" will vary from subject to subject, depending on the age and general condition of the individual, the particular active agent or agents, and the like. Thus, it is not always possible to specify an exact "effective amount". However, an appropriate "effective" amount in any individual case may be determined by one of ordinary skill in the art using routine experimentation.

According to the present invention the dosage of the alpha-2 adrenergic agonist can be reduced when combined with a Sigma ligand of general formula (I), and therefore attaining the same analgesic effect with a reduced dosage, and thus attenuating the adverse effects.

For example, the dosage regime that must be administered to the patient will depend on the patient's weight, the type of application, the condition and severity of the disease. A preferred dosage regime comprises an administration of a Sigma compound of general formula (I) within a range of 0.5 to 100 mg/kg and of the alpha-2 adrenergic agonist from 0.15 to 15 mg/kg. The administration may be performed once or in several occasions.

Having described the present invention in general terms, it will be more easily understood by reference to the following examples which are presented as an illustration and are not intended to limit the present invention.

__Examples__

**Example 1. Synthesis of 4-{2-[5-methyl-1-(naphthalen-2-yl)-1H-pyrazol-3-yloxy]ethyl}morpholine (compound 63) and its hydrochloride salt**

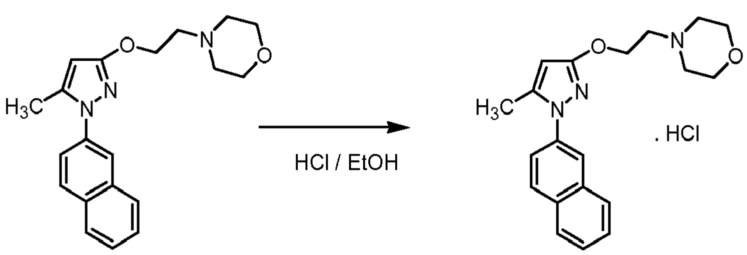


Compound 63 Compound 63·HCl

Compound 63 can be can be prepared as disclosed in the previous application WO2006/021462. Its hydrochloride can be obtained according the following procedure:

Compound 63 (6.39 g) was dissolved in ethanol saturated with HCl, The mixture was stirred then for some minutes and evaporated to dryness. The residue was crystallized from isopropanol. The mother liquors from the first crystallization afforded a second crystallization by concentrating. Both crystallizations taken together yielded 5.24 g (63 %) of the corresponding hydrochloride salt (m.p. = 197-199°C).

$^1$H-NMR (DMSO-$d_6$) $\delta$ ppm: 10,85 (bs, 1 H), 7,95 (m, 4H), 7,7 (dd, J=2,2, 8,8 Hz, 1 H), 7,55 (m, 2H), 5,9 (s, 1 H), 4,55 (m, 2H), 3,95 (m, 2H), 3,75 (m, 2H), 3,55-3,4 (m, 4H), 3,2 (m, 2H), 2,35 (s, 3H).

HPLC purity: 99.8%

Two models of nocicepcion were used, the tail-flick test and the hot-plate test, both in mice.

**Example 2. Tail-flick**

Tail-flick test is a classical model of acute thermal pain (D'Amour and Smith, 1941) Pain sensitivity is measured in response to a radiant heat beam focused on the mouse tail. Latency to the first pain response is recorded as a measure of thermal pain sensitivity. The withdrawal is a classic nocifensive reflex that removes the body apart from the source of pain.

***Experimental conditions***

Male Swiss CD-1 mice weighing between 30 and 35 g (Charles River, Barcelona, Spain) were used for all the experiments. The drugs evaluated were compound 63·HCl and clonidine hydrochloride and guanfacine, as alpha-2 adrenergic agonists. Compound 63·HCl was injected intraperitoneally (i.p.) in a volume of 10 ml/kg in 0.5% hydroxypropyl methyl cellulose (HPMC) and clonidine or guanfacine were administered subcutaneously (s.c.) dissolved in isotonic saline (0.9% NaCl) solution, in a volume of 5 ml/kg. Control animals received the same volume of vehicle.

Antinociception was assessed using the radiant heat tail-flick test as described by Moncada et al., 2003. Briefly, the animals were restrained in a Plexiglas tube and placed on the tail-flick apparatus. The radiant heat source was focused on the proximal portion of the tail at about 7.5 cm of the tip. Vehicle tail-flick latency (VL) was determined using a stimulus intensity adjusted to elicit a mean reaction time in the range of 2-4 s. Following vehicle or compound administration, test latencies (TL) were obtained at 30 min. A cut off time of 10 s was employed in order to prevent tissue damage. All experiments were performed under blind conditions.

Data were expressed as means $\pm$S.E.M of the paw withdrawal latency (s.).

In order to generate dose-response curves, raw data were converted to % maximum possible antinociceptive effect using the following equation:

$$\% \text{ MPE} = [(\text{TL}-\text{VL}) / (\text{Cut off}-\text{VL})] \cdot 100$$

where MPE: maximum possible antinociceptive effect; TL: test latencies and VL: vehicle latency. The number of animals in each group is given in figure legends.

***2.1. Effects of the compound 63·HCl alone or combined with the alpha-2 agonist clonidine***

Intraperitoneal administration of compound 63·HCl (5, 10, and 40 mg/kg) failed to produce a statistically significant antinociceptive effect (Figure 1). On the contrary, the combination of all these doses with subcutaneous administration of 0.125 mg/kg of clonidine produced significant antinociception. Moreover, the combination of 40 mg/kg compound 63·HCl + clonidine was significantly better than clonidine alone.

***2.2. Effects of the alpha-2 agonist clonidine alone or combined with the compound 63·HCl.***

Groups of mice were injected with several doses of clonidine subcutaneously (0.125, 0.25, 0.5 and 1 mg/kg). A selected dose without antinociceptive effect of compound 63·HCl (40 mg/kg, ip) was combined with those of clonidine.

Figure 2 represents the effect of clonidine alone and combined with compound 63·HCl in the tail-flick test. Compound 63·HCl potentiates the antinociceptive effect of clonidine, as evidenced by the displacement to the left of the dose-response curve. The 50% effective dose of clonidine was significantly smaller when combined with compound 63·HCl compared with clonidine alone (ED50=0.063 mg/kg versus ED50= 0.18 mg/kg).

***2.3. Effects of the alpha-2 agonist guanfacine alone or combined with the Sigma ligand Compound 63·HCl.***

Figure 3 shows that guanfacine at a dose of 1.25 mg/kg, s.c. is not effective compared to vehicle, but when combined with 40 mg/kg Compound 63·HCl, a significant analgesic effect compared to vehicle and to guanfacine alone is obtained.

**Example 3. Hot plate**

The hot plate is another classical test of acute thermal nociception. This test consists of introducing mice into an open-ended cylindrical space with a floor consisting of a metallic plate that is heated to a constant temperature. Typical behavioural responses in mice are paw-licking and jumping (Le Bars et al., 2001)

***Experimental conditions***

Male Swiss CD-1 mice weighing between 30 and 35 g (Charles River, Barcelona, Spain) were used for all the experiments. The drugs evaluated were the Sigma ligand Compound 63·HCl and the alpha-2 adrenergic agonists clonidine, guanfacine, and dexmedetomidine. Compound 63·HCl was injected intraperitoneally (i.p.) in a volume of 10 ml/kg in 0.5% hydroxypropyl methyl cellulose (HPMC), whereas clonidine, guanfacine and dexmedetomidine were administrated subcutaneously (s.c.) in saline, in a volume of 5 ml/kg. Control animals received the same volume of vehicle. The hot plate test was done 30 min after compound's administration. Mice were placed individually on the hot-plate at 50°C and the reaction time starting from the placement of the mouse on the hot plate to the time of hind paw licking (HPL) was measured with a stopwatch. An independent untreated group of animals from each strain was used to evaluate the basal latencies and five times those values were chosen as the cut-off time to avoid tissue damage.

Data were expressed as means ±S.E.M of the paw withdrawal latency (s).

In order to generate dose-response curves, raw data were converted to % maximum possible antinociceptive effect using the following equation:

$$\% \text{ MPE} = [(\text{TL-VL}) / (\text{Cut off-VL})] \cdot 100$$

where MPE: maximum possible antinociceptive effect; TL: test latencies and VL: vehicle latency. The number of animals in each group is given in figure legends.

***3.1. Effect of the Sigma ligands Compound 63·HCl alone or combined with the alpha-2 agonist clonidine***

Intraperitoneal administration of Compound 63·HCl (40 mg/kg) failed to produce a statistically significant antinociceptive effect (Figure 4). However, the combination of this compound with subcutaneous administration of 0.25 mg/kg clonidine produced significant antinociception. Moreover, its combination with clonidine was significantly better than clonidine alone.

*3.2.* ***Effects of the alpha-2 agonist clonidine alone or combined with the Sigma ligand Compound 63·HCl.***

Groups of mice were injected with various doses of clonidine subcutaneously (0.125, 0.25, 0.5 and 1 mg/kg). A selected dose without antinociceptive effect of Sigma ligand Compound 63·HCl (40 mg/kg, ip) was combined with those of clonidine.

Figure 5 represents the effect of clonidine alone and combined with the Sigma ligand Compound 63·HCl in the hot-plate test. Compound 63·HCl alone showed no activity, but it potentiated the antinociceptive effect of clonidine, as evidenced by the displacement to the left of the dose-response curve. The 50% effective dose of clonidine was significantly smaller when combined with Compound 63·HCl compared with clonidine alone (ED50=0.11 mg/kg versus ED50= 0.30 mg/kg).

***3.3. Effects of the alpha-2 agonists guanfacine and dexmedetomidine alone or combined with the Sigma ligand Compound 63·HCl.***

Figure 6 shows that guanfacine and dexmedetomidine doses of 5 and 0.01 mg/kg, s.c., respectively are not effective compared to control group, but when combined with 40 mg/kg Compound 63·HCl, a significant analgesic effect compared to control is obtained.

**References:**

Asano, T., Dohi, S., Ohta, S., Shimonaka, H., Lida, H. Antinociception by epidural and systemic alpha(2)-adrenoceptor agonists and their binding affinity in rat spinal cord and brain, Anesth Analg. 2000; 90 (2): 400-407.

Bowen W.D.; Pharmaceutica Acta Helvetiae; 2000; 74: 211-218.

Buerkle, H., Yaksh, T.L. Pharmacological evidence for different alpha 2-adrenergic receptor sites mediating analgesia and sedation in the rat, Br J Anaesth. 1998; 81 (2): 208-215.

Cobos, E.J., Entrena, J.M., Nieto, F.R., Cendan, C.M., Del Pozo, E. Pharmacology and therapeutic potential of Sigma(1) receptor ligands. Curr.Neuropharmacol. 2008; 6, 344-366.

D'Amour, F.E. and Smith, D.L. A method for determining the loss of pain sensation, J. Pharmacol. Exp. Ther. 1941; 72: 74-79.

Dias, V.C., Tendler, B., Oparil, S., Reilly, P.A., Snar, P.,White, W.B. Clinical experience with transdermal clonidine in African-American and Hispanic-American patients with hypertension: evaluation from a 12-week prospective, open-label clinical trial in community-based clinics, Am J Ther. 1999; 6 (1): 19-24.

Eisenach, J.C., Hood, D.D., Curry, R. Intrathecal, but not intravenous, clonidine reduces experimental thermal or capsaicin-induced pain and hyperalgesia in normal volunteers; Anesth Analg; 1998; 87: 591-596.

Gentili, M., Houssel, P., Osman, M., Henel, D.,Juhel, A., Bonnet, F. Intra-articular morphine and clonidine produce comparable analgesia but the combination is not more effective, Br J Anaesth. 1997; 79 (5): 660-661.

Hall, J.E., Uhrich, T.D., Ebert, T.J. Sedative, analgesic and cognitive effects of clonidine infusions in humans, Br J Anaesth. 2001; 86 (1): 5-11.

Kaiser et al.; Neurotransmissions; 1991; 7(1); 1-5.

Le Bars, D., Gozariu, M., Cadden, S.W. Animal models of nociception. Pharmacol.Rev. 2001; 53, 597-652.

Maurice, T., Su, T.P., The pharmacology of Sigma-1 receptors. Pharmacol.Ther. 2009; 124, 195-206.

Merskey et al.; IASP, Classification of Chronic pain; 2nd Ed.; IASP Press (2002); 210-213.

Moncada A, Cendán CM, Baeyens JM, Del Pozo E. Effects of serine/threonine protein phosphatase inhibitors on morphine-induced antinociception in the tail flick test in mice. Eur J Pharmacol. 2003; Mar 28; 465(1-2): 53-60.

Nakajima K, Obata H, Iriuchijima N, Saito S. An increase in spinal cord noradrenaline is a major contributor to the antihyperalgesic effect of antidepressants after peripheral nerve injury in the rat. Pain. 2012; 153(5): 990-7.

Ongioco, C.D. Richardson, X.L. Rudner, M. Stafford-Smith and D.A. Schwinn. Alpha2-adrenergic receptors in human dorsal root ganglia: predominance of alpha2b and alpha2c subtype mRNSs, Anesthesiology 2000; 92 (4): 968-976.

Puskas, F., Camporesi, E.M., O'Leary, C.E., Hauser, M., Nasrallah, F.V. Intrathecal clonidine and severe hypotension after cardiopulmonary bypass, Anesth Analg. 2003; 97 (5): 1251-1253.

Romero, L., Zamanillo, D., Nadal, X., Sanchez-Arroyos, R., Rivera-Arconada, I., Dordal, A., Montero, A., Muro, A., Bura, A., Segales, C., Laloya, M., Hernandez, E., Portillo-Salido, E., Escriche, M., Codony, X., Encina, G., Burgueno, J., Merlos, M., Baeyens, J., Giraldo, J., Lopez-Garcia, J., Maldonado, R., Plata-Salaman, C., Vela, J. Pharmacological properties of S1RA, a new Sigma-1 receptor antagonist that inhibits neuropathic pain and activity-induced spinal sensitization. Br.J.Pharmacol. 2012; doi: 10.1111/j.1476-5381.

Ronsisvalle, G. et al.; Pure Appl. Chem.; 2011; 73; 1499-1509.

E. Samso, E., Valles, J., Pol, O., Gallart, L., Puig, M.M.. Comparative assessment of the anaesthetic and analgesic effects of intramuscular and epidural clonidine in humans, Can J Anaesth. 1996; 43 (12): 1195-1202.

Sierralta, F., Naquira, D., Pinardi, G., Miranda, H.F. Alpha-Adrenoceptor and opioid receptor modulation of clonidine-induced antinociception, Br J Pharmacol. 1996; 119 (3): 551-554.

Snyder, S.H., Largent, B.L. J. Neuropsychiatry; 1989, 1, 7.

S.G. Wilson, S.G., Smith, S.B., Chesler, E.J., Melton, K.A., Haas, J.J., Mitton, B., Strasburg, K., Hubert, L., Rodriguez-Zas, S.L., Mogil, J.S.. The heritability of antinociception: common pharmacogenetic mediation of five neurochemically distinct analgesics, J Pharmacol Exp Ther. 2003; 304 (2): 547-559.

Walker, J.M. et al., Pharmacological Reviews, 1990, 42, 355.

Yaksh, T.L. Pharmacology of spinal adrenergic systems which modulate spinal nociceptive processing. Pharmacol Biochem Behav. 1985; 22(5): 845-58.

## Claims

**1.** A combination of at least one Sigma ligand and at least one alpha-2-adrenergic agonist ligand, for simultaneous, separate or sequential administration, wherein the Sigma ligand has a general formula (I)

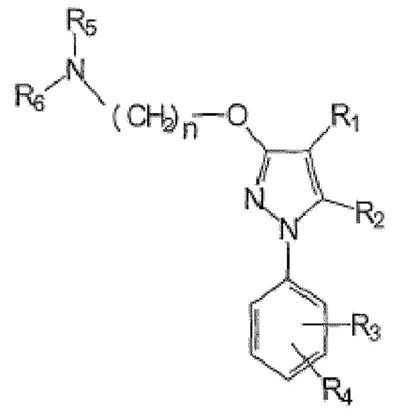

**(I)**

wherein,

**$R_1$** is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, $-COR_8$, $-C(O)OR_8$, $-C(O)NR_8R_9$, $-CH{=}NR_8$, -CN, $-OR_8$, $-OC(O)R_8$, $-S(O)_t-R_8$, $-NR_8R_9$, $-NR_8C(O)R_9$, $-NO_2$, $-N{=}CR_8R_9$, and halogen;

**$R_2$** is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, $-COR_8$, $-C(O)OR_8$, $-C(O)NR_8R_9$, $-CH{=}NR_8$, -CN, $-OR_8$, $-OC(O)R_8$, $-S(O)_t-R_8$, $-NR_8R_9$, $-NR_8C(O)R_9$, $-NO_2$, $-N{=}CR_8R_9$, and halogen;

**$R_3$** and **$R_4$** are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, $-COR_8$, $-C(O)OR_8$, $-C(O)NR_8R_9$, $-CH{=}NR_8$, -CN, $-OR_8$, $-OC(O)R_8$, $-S(O)_t-R_8$, $-NR_8R_9$, $-NR_8C(O)R_9$, $-NO_2$, $-N{=}CR_8R_9$, and halogen, or together with the phenyl they form an optionally substituted fused ring system;

**$R_5$** and **$R_6$** are independently selected from the group consisting of hydrogen, substituted or unsubstituted alkyl, substituted or unsubstituted cycloalkyl,
substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, - $COR_8$, $-C(O)OR_8$, $-C(O)NR_8R_9$, $-CH=NR_8$, -CN, $-OR_8$, $-OC(O)R_8$, $-S(O)_t-R_8$, - $NR_8R_9$, $-NR_8C(O)R_9$, $-NO_2$, $-N=CR_8R_9$, and halogen;
or together form, with the nitrogen atom to which they are attached, a substituted or unsubstituted, aromatic or non-aromatic heterocyclyl group;
**n** is selected from 1, 2, 3, 4, 5, 6, 7 and 8;
**t** is 0, 1, or 2;
**$R_8$** and **$R_9$** are each independently selected from hydrogen, substituted or unsubstituted alkyl, substituted or unsubstituted cycloalkyl, substituted or unsubstituted alkenyl, substituted or unsubstituted aryl, substituted or unsubstituted, aromatic or non-aromatic heterocyclyl, substituted or unsubstituted alkoxy, substituted or unsubstituted aryloxy, and halogen.

**2.** The combination according to claim 1, wherein $R_1$ is selected from H, $-COR_8$, and substituted or unsubstituted alkyl.

**3.** The combination according to claim 1 or 2, wherein $R_2$ is H or alkyl.

**4.** The combination according to any one of the preceding claims, wherein $R_3$ and $R_4$ together with the phenyl group form a naphthyl ring system.

**5.** The combination according to any one of the preceding claims, wherein n is selected from 2, 3, and 4.

**6.** The combination according to any one of the preceding claims, wherein $R_5$ and $R_6$ together form a morpholine-4-yl group.

**7.** The combination according to claim 1, wherein the Sigma ligand of general formula (I) is selected from:

[1] 4-{2-(1-(3,4-dichlorophenyl)-5-methyl-1H pyrazol-3-yloxy)ethyl} morpholine,
[2] 2-[1-(3,4-Dichlorophenyl)-5-methyl-1H-pyrazol-3-yloxy]-N,N-diethylethanamine,
[3] 1-(3,4-Dichlorophenyl)-5-methyl-3-[2-(pyrrolidin-1-yl)ethoxy]-1H-pyrazole,
[4] 1-(3,4-Dichlorophenyl)-5-methyl-3-[3-(pyrrolidin-1-yl)propoxy]-1H-pyrazole,
[5] 1-{2-[1-(3,4-Dichlorophenyl)-5-methyl-1H-pyrazol-3-yloxy]ethyl}piperidine,
[6] 1-{2-[1-(3,4-dichlorophenyl)-5-methyl-1H-pyrazol-3-yloxy]ethyl}-1H-imidazole,
[7] 3-{1-[2-(1-(3,4-Dichlorophenyl)-5-methyl-1H-pyrazol-3-yloxy)ethyl]piperidin-4-yl}-3H-imidazo[4,5-b]pyridine,
[8]1-{2-[1-(3,4-Dichlorophenyl)-5-methyl-1H-pyrazol-3-yloxy]ethyl}-4-methylpiperazine,
[9] Ethyl 4-{2-[1-(3,4-dichlorophenyl)-5-methyl-1H-pyrazol-3-yloxy]ethyl} piperazine carboxylate,
[10] 1-(4-(2-(1-(3,4-dichlorophenyl)-5-methyl-1H-pyrazol-3-yloxy)ethyl)piperazin-1-yl)ethanone,
[11] 4-{2-[1-(4-Methoxyphenyl)-5-methyl-1H-pyrazol-3-yloxy]ethyl}morpholine,
[12] 1-(4-Methoxyphenyl)-5-methyl-3-[2-(pyrrolidin-1-yl)ethoxy]-1H-pyrazole,
[13] 1-(4-Methoxyphenyl)-5-methyl-3-[3-(pyrrolidin-1-yl)propoxy]-1H-pyrazole,
[14] 1-[2-(1-(4-Methoxyphenyl)-5-methyl-1H-pyrazol-3-yloxy)ethyl]piperidine,
[15]1-{2-[1-(4-Methoxyphenyl)-5-methyl-1H-pyrazol-3-yloxy]ethyl}-1H-imidazole,
[16] 4-{2-[1-(3,4-Dichlorophenyl)-5-phenyl-1H-pyrazol-3-yloxy]ethyl} morpholine,
[17] 1-(3,4-Dichlorophenyl)-5-phenyl-3-[2-(pyrrolidin-1-yl)ethoxy]-1H-pyrazole,
[18] 1-(3,4-Dichlorophenyl)-5-phenyl-3-[3-(pyrrolidin-1-yl)propoxy]-1H-pyrazole,
[19] 1-{2-[1-(3,4-Dichlorophenyl)-5-phenyl-1H-pyrazol-3-yloxy]ethyl}piperidine,
[20] 1-{2-[1-(3,4-Dichlorophenyl)-5-phenyl-1H-pyrazol-3-yloxy]ethyl}-1H-imidazole,
[21]2-{2-[1-(3,4-dichlorophenyl)-5-phenyl-1H-pyrazol-3-yloxy]ethyl}-1,2,3,4-tetrahydroisoquinoline,
[22] 4-{4-[1-(3,4-Dichlorophenyl)-5-methyl-1H-pyrazol-3-yloxy]butyl} morpholine,
[23] 1-(3,4-Dichlorophenyl)-5-methyl-3-[4-(pyrrolidin-1-yl)butoxy]-1H-pyrazole,
[24] 1-{4-[1-(3,4-Dichlorophenyl)-5-methyl-1H-pyrazol-3-yloxy]butyl}piperidine,
[25]1-{4-[1-(3,4-Dichlorophenyl)-5-methyl-1H-pyrazol-3-yloxy]butyl}-4-methylpiperazine,
[26] 1-{4-[1-(3,4-Dichlorophenyl)-5-methyl-1H-pyrazol-3-yloxy]butyl}-1H-imidazole,
[27] 4-[1-(3,4-Dichlorophenyl)-5-methyl-1H-pyrazol-3-yloxy]-N,N-diethylbutan-1-amine,
[28]1-{4-[1-(3,4-dichlorophenyl)-5-methyl-1H-pyrazol-3-yloxy]butyl}-4-phenylpiperidine,

[29] 1-{4-[1-(3,4-dichlorophenyl)-5-methyl-1H-pyrazol-3-yloxy]butyl}-6,7-dihydro-1H-indol-4(5H)-one,
[30] 2-{4-[1-(3,4-dichlorophenyl)-5-methyl-1H-pyrazol-3-yloxy]butyl}-1,2,3,4-tetrahydroisoquinoline,
[31] 4-{2-[1-(3,4-dichlorophenyl)-5-isopropyl-1H-pyrazol-3-yloxy]ethyl} morpholine,
[32]2-[1-(3,4-Dichlorophenyl)-5-isopropyl-1H-pyrazol-3-yloxy]-N,N-diethylethanamine,
[33] 1-(3,4-Dichlorophenyl)-5-isopropyl-3-[2-(pyrrolidin-1-yl)ethoxy]-1H-pyrazole,
[34] 1-(3,4-Dichlorophenyl)-5-isopropyl-3-[3-(pyrrolidin-1-yl)propoxy]-1H-pyrazole,
[35] 1-{2-[1-(3,4-Dichlorophenyl)-5-isopropyl-1H-pyrazol-3-yloxy]ethyl} piperidine,
[36] 2-{2-[1-(3,4-dichlorophenyl)-5-isopropyl-1H-pyrazol-3-yloxy]ethyl}-1,2,3,4-tetrahydroisoquinoline,
[37] 4-{2-[1-(3,4-dichlorophenyl)-1H-pyrazol-3-yloxy]ethyl}morpholine,
[38] 2-[1-(3,4-dichlorophenyl)-1H-pyrazol-3-yloxy] N,N-diethylethanamine,
[39] 1-(3,4-dichlorophenyl)-3-[2-(pyrrolidin-1-yl)ethoxy]-1H-pyrazole,
[40] 1-{2-[1-(3,4-dichlorophenyl)-1H-pyrazol-3-yloxy]ethyl}piperidine,
[41] 1-(3,4-dichlorophenyl)-3-[3-(pyrrolidin-1-yl)propoxy]-1H-pyrazole,
[42] 1-{2-[1-(3,4-Dichlorophenyl)-5-methyl-1H-pyrazol-3-yloxy]ethyl}piperazine,
[43] 1-{2-[1-(3,4-Dichlorophenyl)-5-methyl-1H-pyrazol-3-yloxy]ethyl}pyrrolidin-3-amine,
[44] 4-{2-[1-(3,4-Dichlorophenyl)-4,5-dimethyl-1H-pyrazol-3-yloxy]ethyl} morpholine,
[46]2-[1-(3,4-Dichlorophenyl)-4,5-dimethyl-1H-pyrazol-3-yloxy]-N,N-diethylethanamine,
[47] 1-(3,4-Dichlorophenyl)-4,5-dimethyl-3-[2-(pyrrolidin-1-yl)ethoxy]-1H-pyrazole,
[48] 1-(3,4-Dichlorophenyl)-4,5-dimethyl-3-[3-(pyrrolidin-1-yl)propoxy]-1H-pyrazole,
[49] 1-{2-[1-(3,4-Dichlorophenyl)-4,5-dimethyl-1H-pyrazol-3-yloxy]ethyl} piperidine,
[50] 4-{4-[1-(3,4-dichlorophenyl)-1H-pyrazol-3-yloxy]butyl}morpholine,
[51]((2S,6R)-4-{4-[1-(3,4-dichlorophenyl)-1H-pyrazol-3-yloxy]butyl}-2,6-dimethylmorpholine,
[52] 1-{4-[1-(3,4-Dichlorophenyl)-1H-pyrazol-3-yloxy]butyl}piperidine,
[53] 1-(3,4-Dichlorophenyl)-3-[4-(pyrrolidin-1-yl)butoxy]-1H-pyrazole,
[55] 4-[1-(3,4-dichlorophenyl)-1H-pyrazol-3-yloxy]-N,N-diethylbutan-1-amine,
[56] N-benzyl-4-[1-(3,4-dichlorophenyl)-1H-pyrazol-3-yloxy]-N-methylbutan-1-amine,
[57]4-[1-(3,4-dichlorophenyl)-1H-pyrazol-3-yloxy]-N-(2-methoxyethyl)-N-methylbutan-1-amine,
[58] 4-{4-[1-(3,4-dichlorophenyl)-1H-pyrazol-3-yloxy]butyl}thiomorpholine,
[59]1-[1-(3,4-Dichlorophenyl)-5-methyl-3-(2-morpholinoethoxy)-1H-pyrazol-4-yl]ethanone,
[60]1-{1-(3,4-dichlorophenyl)-5-methyl-3-[2-(pyrrolidin-1-yl)ethoxy]-1H-pyrazol-4-yl}ethanone,
[61] 1-{1-(3,4-dichlorophenyl)-5-methyl-3-[2-(piperidin-1-yl)ethoxy]-1H-pyrazol-4-yl}ethanone,
[62] 1-{1-(3,4-dichlorophenyl)-3-[2-(diethylamino)ethoxy]-5-methyl-1H-pyrazol-4-yl}ethanone,
[63] 4-{2-[5-Methyl-1-(naphthalen-2-yl)-1H-pyrazol-3-yloxy]ethyl}morpholine,
[64] N,N-Diethyl-2-[5-methyl-1-(naphthalen-2-yl)-1H-pyrazol-3-yloxy] ethanamine,
[65] 1-{2-[5-Methyl-1-(naphthalen-2-yl)-1H-pyrazol-3-yloxy]ethyl}piperidine, and
[66] 5-Methyl-1-(naphthalen-2-yl)-3-[2-(pyrrolidin-1-yl)ethoxy]-1H-pyrazole,
or a pharmaceutically acceptable salt, solvate or prodrug thereof.

**8.** The combination according to any one of the preceding claims, wherein the alpha-2 adrenergic agonist ligand is selected from the group consisting of imino-imidazolines, imidazolines, imidazoles, azepines, thiazines, oxazolines, guanidines, catecholamines, derivatives thereof and mixtures thereof.

**9.** The combination according to claim 8, wherein the alpha-2 adrenergic agonist ligand is selected from the group consisting of clonidine, apraclonidine, tizanidine, naphazoline, xymetazoline, tetrahydrozoline, tramazoline, fadolmidine, detomidine, medetomidine, dexmedetomidine, B-HT 920 (6-allyl-2-amino-5,6,7,8 tetrahydro-4H-thiazolo[4,5-d]-azepine) and B-HT 933, xylazine, rilmenidine, guanabenz, guanoxabenz, guanfacine, guanethidine, and methyldopa.

**10.** The combination according to any one of claims 8 to 9, wherein the alpha-2 adrenergic agonist ligand is selected from the group consisting of clonidine, tizanidine, guanfacine and dexmedetomidine.

**11.** The combination according to any one of the preceding claims, wherein the combination comprises 4-{2-[5-methyl-1-(naphthalen-2-yl)-1H-pyrazol-3-yloxy]ethyl}morpholine and clonidine, tizanidine, guanfacine or dexmedetomidine.

**12.** The combination according to any one of the preceding claims, wherein the combination comprises 4-{2-[5-methyl-1-(naphthalen-2-yl)-1H-pyrazol-3-yloxy]ethyl}morpholine hydrochloride and clonidine, tizanidine, guanfacine or dexmedetomidine.

**13.** The combination according to any one of the preceding claims for use in the prophylaxis and/or treatment of pain.

**14.** The combination according to claim 13 wherein the pain is selected from peripheral neuropathic pain, allodynia, causalgia, hyperalgesia, hyperesthesia, hyperpathia, neuralgia, neuritis and neuropathy.

**15.** Sigma ligand of general formula (I) as defined in any one of claims 1 to 7, or a pharmaceutically acceptable salt, isomer, prodrug or solvate thereof, for use in potentiating the analgesic effect of an alpha-2 adrenergic agonist when said alpha-2 adrenergic agonist is used in the prophylaxis and/or treatment of pain.

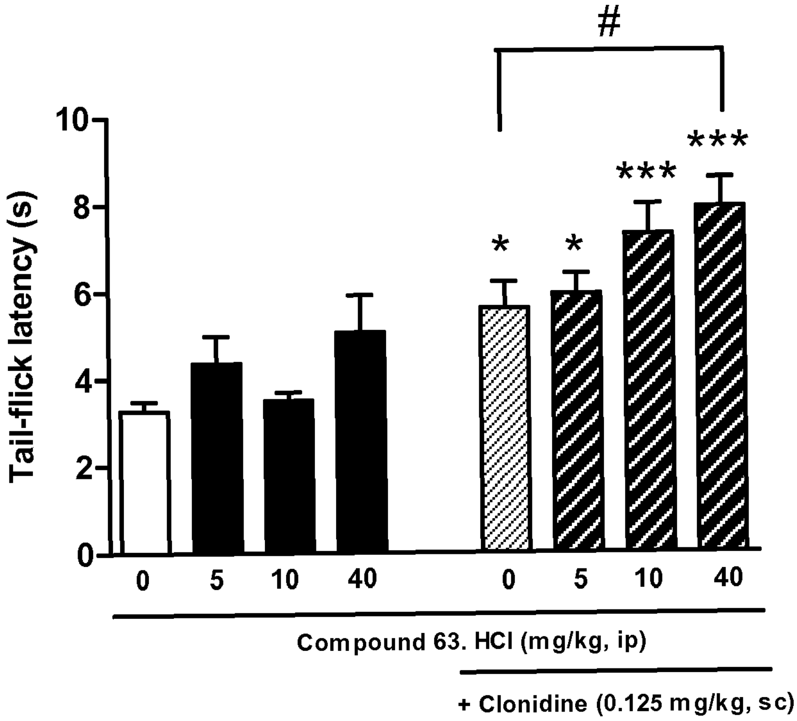
#
10
8
6
4
2
0
Tail-flick latency (s)
*
*
***
***
0
5
10
40
0
5
10
40
Compound 63. HCl (mg/kg, ip)
+ Clonidine (0.125 mg/kg, sc)

Fig. 1

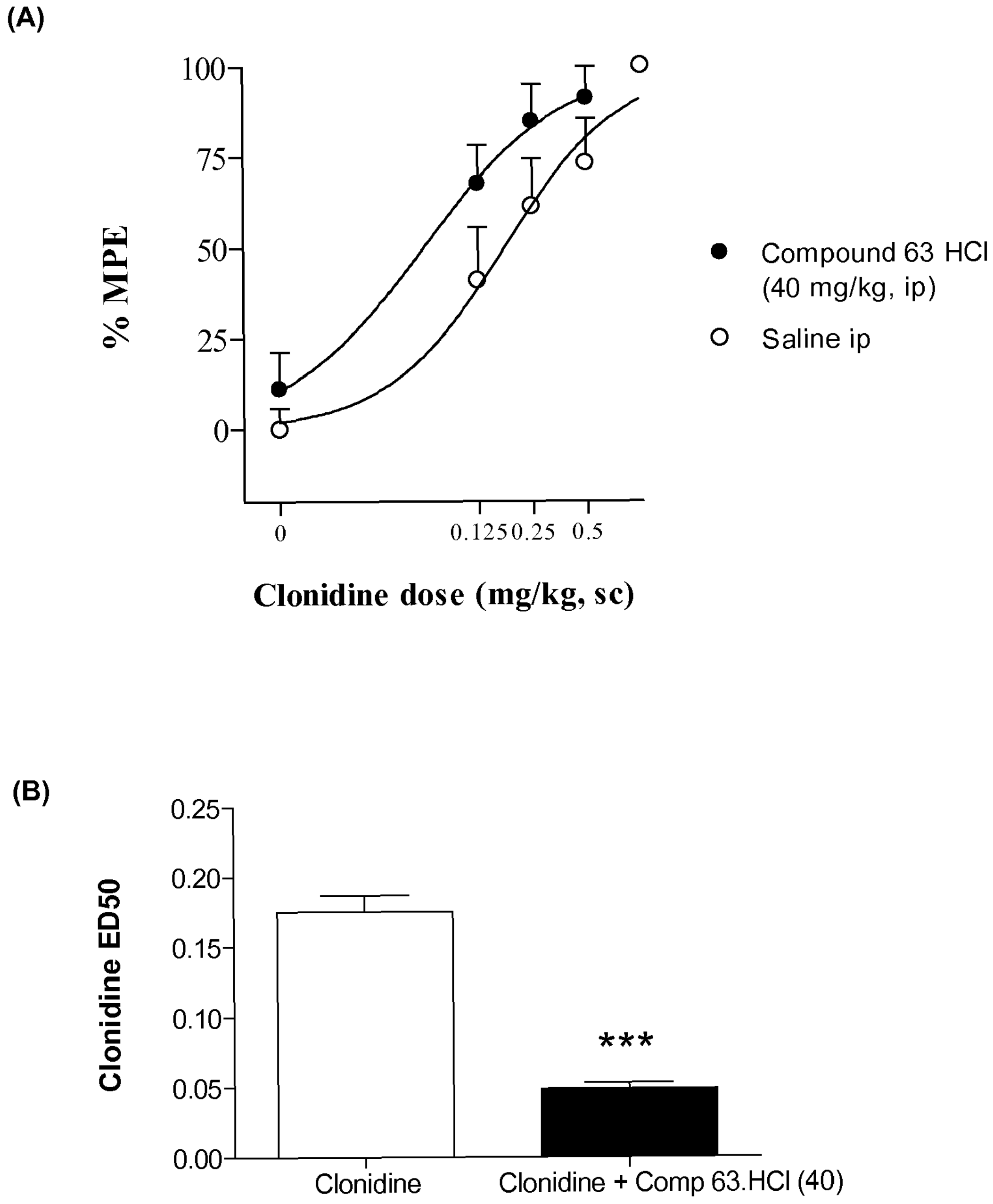
(A)
100
75
50
25
0
% MPE
0
0.125
0.25
0.5
Clonidine dose (mg/kg, sc)
Compound 63 HCl
(40 mg/kg, ip)
Saline ip
(B)
0.25
0.20
0.15
0.10
0.05
0.00
Clonidine ED50
***
Clonidine
Clonidine + Comp 63.HCl (40)

Fig. 2

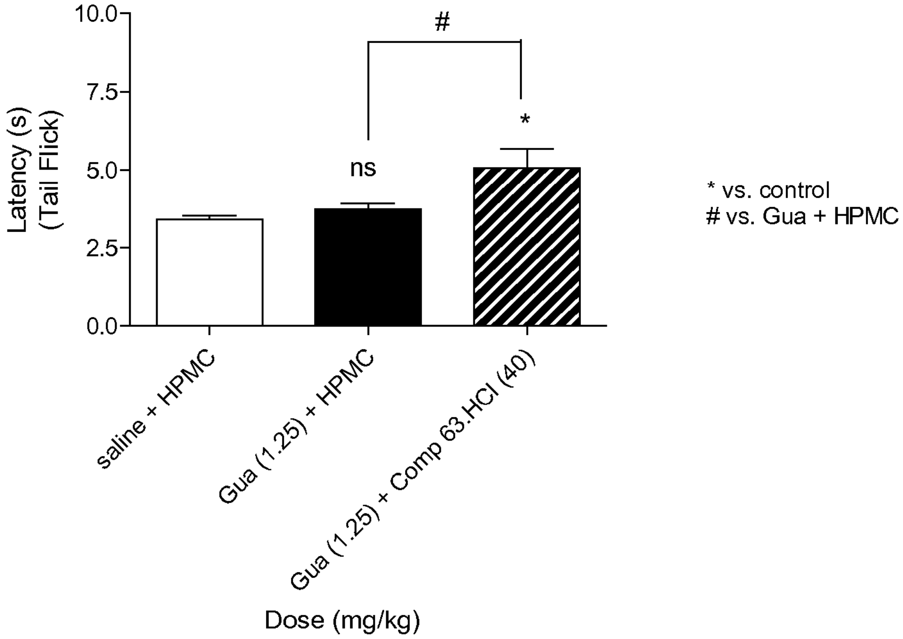
10.0
7.5
5.0
2.5
0.0
Latency (s)
(Tail Flick)
#
ns
*
* vs. control
# vs. Gua + HPMC
saline + HPMC
Gua (1.25) + HPMC
Gua (1.25) + Comp 63.HCl (40)
Dose (mg/kg)

**Fig. 3**

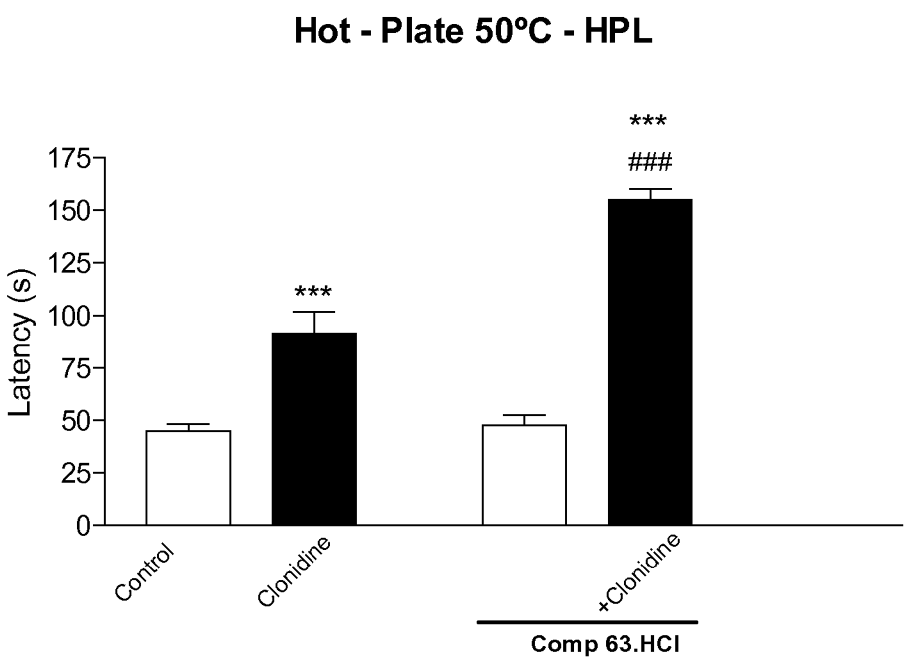
Hot - Plate 50ºC - HPL
175
150
125
100
75
50
25
0
Latency (s)
***
***
###
Control
Clonidine
+Clonidine
Comp 63.HCl

**Fig. 4**

(A)

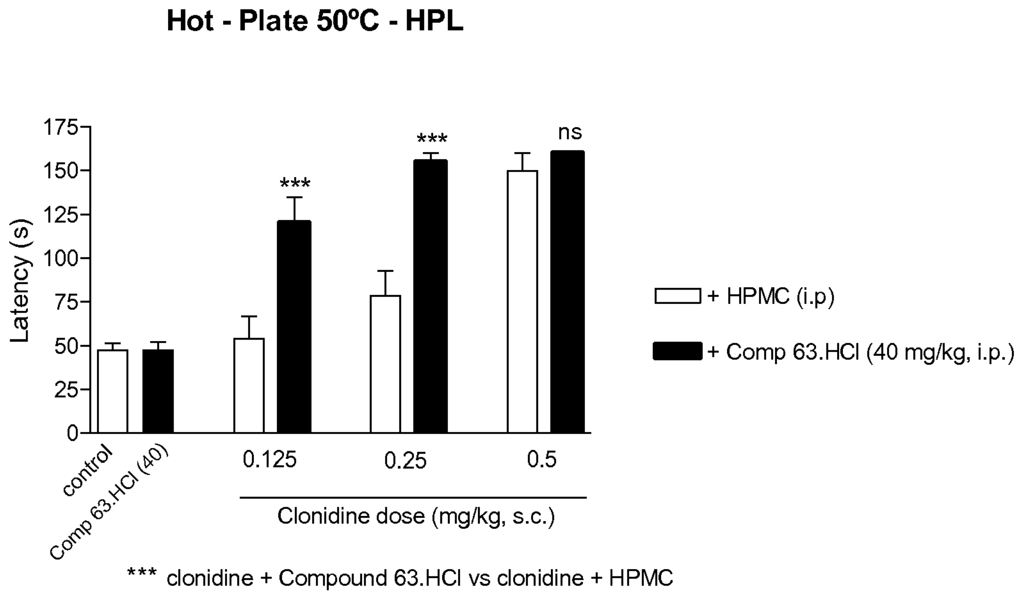
Hot - Plate 50ºC - HPL
Latency (s)
175
150
125
100
75
50
25
0
***
***
ns
control
Comp 63.HCl (40)
0.125
0.25
0.5
Clonidine dose (mg/kg, s.c.)
+ HPMC (i.p)
+ Comp 63.HCl (40 mg/kg, i.p.)
*** clonidine + Compound 63.HCl vs clonidine + HPMC

(B)

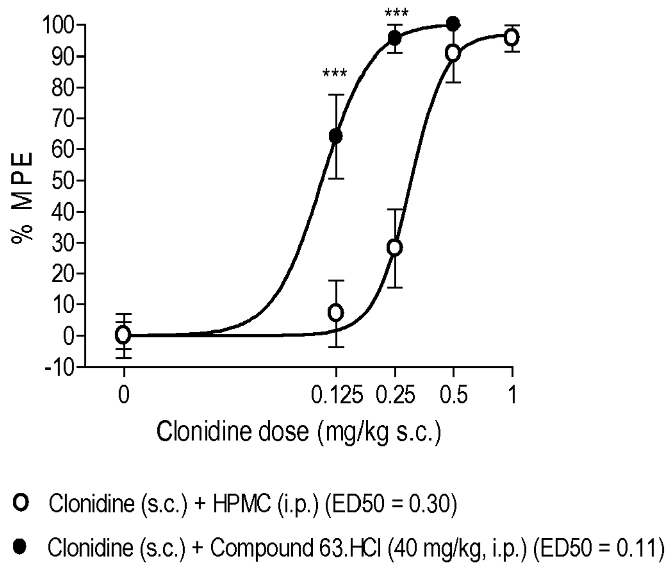
% MPE
100
90
80
70
60
50
40
30
20
10
0
-10
***
***
0
0.125
0.25
0.5
1
Clonidine dose (mg/kg s.c.)
O Clonidine (s.c.) + HPMC (i.p.) (ED50 = 0.30)
● Clonidine (s.c.) + Compound 63.HCl (40 mg/kg, i.p.) (ED50 = 0.11)

Fig. 5

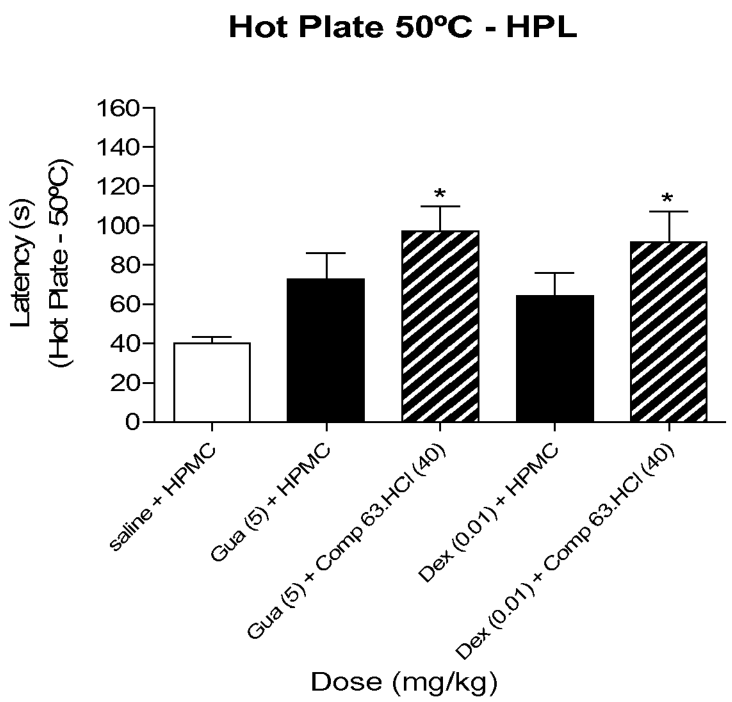
Hot Plate 50ºC - HPL
Latency (s)
(Hot Plate - 50ºC)
160
140
120
100
80
60
40
20
0
*
*
saline + HPMC
Gua (5) + HPMC
Gua (5) + Comp 63.HCl (40)
Dex (0.01) + HPMC
Dex (0.01) + Comp 63.HCl (40)
Dose (mg/kg)

**Fig. 6**

Europäisches Patentamt
European Patent Office
Office européen des brevets

# EUROPEAN SEARCH REPORT

Application Number
EP 13 38 2140

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/046129 A2 (DU PONT [US]; HUGHES KENNETH ANDREW [US]; LAHM GEORGE PHILIP [US]; SEL) 3 June 2004 (2004-06-03) * See the compounds of claim 1 and in particular the specific compounds having the formula: 1070627-08-5, 1070627-41-6, 1070627-43-8, 1070627-44-9, 1070627-45-0, 1070627-46-1, 1070627-47-2, 1070627-48-3, 1070627-49-4, 1070627-50-7, 1070627-51-8, 1070627-52-9, 1070627-53-0, 1070627-54-1, 1070627-55-2, 1070627-56-3, 1070627-57-4, 1070627-58-5, 1070627-59-6, 1070629-17-2, 1070629-18-3, 1070629-19-4, 1070629-20-7, 1070629-21-8, 1070629-22-9, 1070629-23-0, 1070629-24-1, 1070629-25-2, 1070629-26-3, 1070629-27-4, 1070629-28-5, 1070629-29-6, 1070629-30-9, 1070629-31-0, 1070629-32-1, 1070629-33-2, and their combinations with the compound amitraz * ----- | 1,2 | INV. A61K31/155 A61K31/415 A61K31/4168 A61K31/5377 A61P25/06 |
| A | HSU W H ET AL: "Effect of amitraz and chlordimeform on heart rate and pupil diameter in rats: Mediated by alpha2-adrenoreceptors", TOXICOLOGY AND APPLIED PHARMACOLOGY, ACADEMIC PRESS, AMSTERDAM, NL, vol. 73, no. 3, 1 May 1984 (1984-05-01), - 1984, pages 411-415, XP024883603, ISSN: 0041-008X, DOI: 10.1016/0041-008X(84)90093-0 [retrieved on 1984-05-01] * See abstract: amitraz is an alpha-2 adrenergic ligand * ----- | 1,2 | TECHNICAL FIELDS SEARCHED (IPC) A61K |

-/--

The present search report has been drawn up for all claims

3

| Place of search | Date of completion of the search | Examiner |
|---|---|---|
| Munich | 31 May 2013 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 38 2140

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 2 116 539 A1 (ESTEVE LABOR DR [ES]) 11 November 2009 (2009-11-11) * See claims, examples 1-2, figures and see compound N.63: the relevant Sigma ligands of formula (I) in combination with opioid agents, for use in the treatment of pain * ----- | 1,2 | |
| Y | WO 2006/021462 A1 (ESTEVE LABOR DR [ES]; LAGGNER CHRISTIAN [AT]; CUBERES-ALTISENT MARIA R) 2 March 2006 (2006-03-02) * See the claimed compounds for use in the treatment of pain (pages 59-60 and claim 16) * ----- | 1-15 | |
| Y | BERNHARD WÜNSCH: "The [sigma] 1 Receptor Antagonist S1RA Is a Promising Candidate for the Treatment of Neurogenic Pain", JOURNAL OF MEDICINAL CHEMISTRY, vol. 55, no. 19, 11 October 2012 (2012-10-11), pages 8209-8210, XP055064327, ISSN: 0022-2623, DOI: 10.1021/jm3011993 * S1RA, the preferred compound N. 63 of the present application, for use in the treatment of neuropatic pain * ----- | 1-15 | TECHNICAL FIELDS SEARCHED (IPC) |
| | -/-- | | |

The present search report has been drawn up for all claims

3

| Place of search | Date of completion of the search | Examiner |
|---|---|---|
| Munich | 31 May 2013 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 38 2140

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SABETKASAIE M ET AL: "Clonidine and guanfacine-induced antinociception in visceral pain: possible role of alpha2/I2 binding sites", EUROPEAN JOURNAL OF PHARMACOLOGY, ELSEVIER SCIENCE, NL, vol. 501, no. 1-3, 6 October 2004 (2004-10-06), pages 95-101, XP004587598, ISSN: 0014-2999, DOI: 10.1016/J.EJPHAR.2004.08.010 * See page 95, abstract and left hand column: clonidine and guanfacine, the preferred alpha-2 adrenergic analgesic agents of the present application bind also to the imidazoline receptors. These do also play a role in producing an analgesic effect * ----- | 1-15 | |
| Y | SAMPSON CRISTAL ET AL: "Effects of imidazoline I2 receptor ligands on acute nociception in rats.", NEUROREPORT 25 JAN 2012, vol. 23, no. 2, 25 January 2012 (2012-01-25), pages 73-77, XP009169909, ISSN: 1473-558X * See abstract: imidazoline I2 receptor ligands have antinociceptic effect in acute pain * ----- | 1-15 | TECHNICAL FIELDS SEARCHED (IPC) |
| Y | US 2011/269727 A1 (TOLEDANO ANNETTE C [US]) 3 November 2011 (2011-11-03) * See examples and claims: combinations of direct acting alpha-2 adrenergic agents and opioids agents for the treatment of pain * ----- | 1-15 | |

The present search report has been drawn up for all claims

3

| Place of search | Date of completion of the search | Examiner |
|---|---|---|
| Munich | 31 May 2013 | Veronese, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 38 2140

This annex lists the patent family members relating to the patent documents cited in the above-mentioned European search report. The members are as contained in the European Patent Office EDP file on
The European Patent Office is in no way liable for these particulars which are merely given for the purpose of information.

31-05-2013

| Patent document cited in search report | Publication date | Patent family member(s) | Publication date |
|---|---|---|---|
| WO 2004046129 A2 | 03-06-2004 | AT 469143 T | 15-06-2010 |
| | | AU 2003295491 A1 | 15-06-2004 |
| | | BR 0315714 A | 06-09-2005 |
| | | CN 1711255 A | 21-12-2005 |
| | | EP 1560820 A2 | 10-08-2005 |
| | | ES 2342595 T3 | 09-07-2010 |
| | | JP 4648705 B2 | 09-03-2011 |
| | | JP 2006514632 A | 11-05-2006 |
| | | KR 20050075001 A | 19-07-2005 |
| | | MX PA05005025 A | 03-08-2005 |
| | | US 2006014808 A1 | 19-01-2006 |
| | | WO 2004046129 A2 | 03-06-2004 |
| EP 2116539 A1 | 11-11-2009 | AU 2009239968 A1 | 29-10-2009 |
| | | CA 2722345 A1 | 29-10-2009 |
| | | CN 102066334 A | 18-05-2011 |
| | | CO 6410301 A2 | 30-03-2012 |
| | | EC SP10010634 A | 30-12-2010 |
| | | EP 2116539 A1 | 11-11-2009 |
| | | EP 2276744 A1 | 26-01-2011 |
| | | JP 2011518807 A | 30-06-2011 |
| | | KR 20110011640 A | 08-02-2011 |
| | | RU 2010147925 A | 27-05-2012 |
| | | US 2011112095 A1 | 12-05-2011 |
| | | WO 2009130310 A1 | 29-10-2009 |
| WO 2006021462 A1 | 02-03-2006 | AU 2005276590 A1 | 02-03-2006 |
| | | BR PI0514692 A | 17-06-2008 |
| | | CA 2576144 A1 | 02-03-2006 |
| | | EP 1781618 A1 | 09-05-2007 |
| | | EP 2325174 A1 | 25-05-2011 |
| | | JP 5139061 B2 | 06-02-2013 |
| | | JP 2008510767 A | 10-04-2008 |
| | | KR 20070054220 A | 28-05-2007 |
| | | US 2008125416 A1 | 29-05-2008 |
| | | US 2010190780 A1 | 29-07-2010 |
| | | WO 2006021462 A1 | 02-03-2006 |
| US 2011269727 A1 | 03-11-2011 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006021462 A
- WO 2011064296 A1
- WO 2011064315 A1
- WO 2011095579 A1
- WO 2012019984 A1
- WO 2009130310 A1
- WO 2012016980 A2
- WO 2012072782 A1
- WO 2011018487 A1
- WO 2011144721 A1


### Non-patent literature cited in the description


- IASP, Classification of chronic pain. IASP Press, 2002, 210
- **BURGER.** Medicinal Chemistry and Drug Discovery. Wiley, 2001
- Design and Applications of Prodrugs. Harwood Academic Publishers, 1985
- Stedman's Medical Spellchecker. Lippincott Williams & Wilkins, 2006
- **E.W. MARTIN.** *Remington's Pharmaceutical Sciences*
- IASP, Classification of chronic pain. IASP Press, 2002, 213
- IASP, Classification of chronic pain. IASP Press, 2002, 211
- IASP, Classification of chronic pain. IASP Press, 2002, 212
- **ASANO, T. ; DOHI, S. ; OHTA, S. ; SHIMONAKA, H. ; LIDA, H.** Antinociception by epidural and systemic alpha(2)-adrenoceptor agonists and their binding affinity in rat spinal cord and brain. *Anesth Analg.,* 2000, vol. 90 (2), 400-407
- **BOWEN W.D.** *Pharmaceutica Acta Helvetiae,* 2000, vol. 74, 211-218
- **BUERKLE, H. ; YAKSH, T.L.** Pharmacological evidence for different alpha 2-adrenergic receptor sites mediating analgesia and sedation in the rat. *Br J Anaesth.,* 1998, vol. 81 (2), 208-215
- **COBOS, E.J. ; ENTRENA, J.M. ; NIETO, F.R. ; CENDAN, C.M. ; DEL POZO, E.** Pharmacology and therapeutic potential of Sigma(1) receptor ligands. *Curr.Neuropharmacol.,* 2008, vol. 6, 344-366
- **D'AMOUR, F.E. ; SMITH, D.L.** A method for determining the loss of pain sensation. *J. Pharmacol. Exp. Ther.,* 1941, vol. 72, 74-79
- **DIAS, V.C. ; TENDLER, B. ; OPARIL, S. ; REILLY, P.A. ; SNAR, P. ; WHITE, W.B.** Clinical experience with transdermal clonidine in African-American and Hispanic-American patients with hypertension: evaluation from a 12-week prospective, open-label clinical trial in community-based clinics. *Am J Ther.,* 1999, vol. 6 (1), 19-24
- **EISENACH, J.C. ; HOOD, D.D. ; CURRY, R.** Intrathecal, but not intravenous, clonidine reduces experimental thermal or capsaicin-induced pain and hyperalgesia in normal volunteers. *Anesth Analg,* 1998, vol. 87, 591-596
- **GENTILI, M. ; HOUSSEL, P. ; OSMAN, M. ; HENEL, D. ; JUHEL, A. ; BONNET, F.** Intra-articular morphine and clonidine produce comparable analgesia but the combination is not more effective. *Br J Anaesth.,* 1997, vol. 79 (5), 660-661
- **HALL, J.E. ; UHRICH, T.D. ; EBERT, T.J.** Sedative, analgesic and cognitive effects of clonidine infusions in humans. *Br J Anaesth.,* 2001, vol. 86 (1), 5-11
- **KAISER et al.** *Neurotransmissions,* 1991, vol. 7 (1), 1-5
- **LE BARS, D. ; GOZARIU, M. ; CADDEN, S.W.** Animal models of nociception. *Pharmacol.Rev.,* 2001, vol. 53, 597-652
- **MAURICE, T. ; SU, T.P.** The pharmacology of Sigma-1 receptors. *Pharmacol.Ther.,* 2009, vol. 124, 195-206
- **MERSKEY et al.** IASP, Classification of Chronic pain. IASP Press, 2002, 210-213
- **MONCADA A ; CENDÁN CM ; BAEYENS JM ; DEL POZO E.** Effects of serine/threonine protein phosphatase inhibitors on morphine-induced antinociception in the tail flick test in mice. *Eur J Pharmacol.,* 28 March 2003, vol. 465 (1-2), 53-60

- **NAKAJIMA K ; OBATA H ; IRIUCHIJIMA N ; SAITO S.** An increase in spinal cord noradrenaline is a major contributor to the antihyperalgesic effect of antidepressants after peripheral nerve injury in the rat. *Pain,* 2012, vol. 153 (5), 990-7
- **ONGIOCO, C.D. ; RICHARDSON, X.L. ; RUDNER, M. STAFFORD-SMITH ; D.A. SCHWINN.** Alpha2-adrenergic receptors in human dorsal root ganglia: predominance of alpha2b and alpha2c subtype mRNSs. *Anesthesiology,* 2000, vol. 92 (4), 968-976
- **PUSKAS, F. ; CAMPORESI, E.M. ; O'LEARY, C.E. ; HAUSER, M. ; NASRALLAH, F.V.** Intrathecal clonidine and severe hypotension after cardiopulmonary bypass. *Anesth Analg.,* 2003, vol. 97 (5), 1251-1253
- **ROMERO, L. ; ZAMANILLO, D. ; NADAL, X. ; SANCHEZ-ARROYOS, R. ; RIVERA-ARCONADA, I. ; DORDAL, A. ; MONTERO, A. ; MURO, A. ; BURA, A. ; SEGALES, C.** Pharmacological properties of S1RA, a new Sigma-1 receptor antagonist that inhibits neuropathic pain and activity-induced spinal sensitization. *Br.J.Pharmacol.,* 2012
- **RONSISVALLE, G. et al.** *Pure Appl. Chem.,* 2011, vol. 73, 1499-1509
- **E. SAMSO, E. ; VALLES, J. ; POL, O. ; GALLART, L. ; PUIG, M.M.** Comparative assessment of the anaesthetic and analgesic effects of intramuscular and epidural clonidine in humans. *Can J Anaesth.,* 1996, vol. 43 (12), 1195-1202
- **SIERRALTA, F. ; NAQUIRA, D. ; PINARDI, G. ; MIRANDA, H.F.** Alpha-Adrenoceptor and opioid receptor modulation of clonidine-induced antinociception. *Br J Pharmacol.,* 1996, vol. 119 (3), 551-554
- **SNYDER, S.H. ; LARGENT, B.L.** *J. Neuropsychiatry,* 1989, vol. 1, 7
- **S.G. WILSON ; S.G., SMITH, S.B. ; CHESLER, E.J. ; MELTON, K.A. ; HAAS, J.J. ; MITTON, B. ; STRASBURG, K. ; HUBERT, L. ; RODRIGUEZ-ZAS, S.L. ; MOGIL, J.S.** The heritability of antinociception: common pharmacogenetic mediation of five neurochemically distinct analgesics. *J Pharmacol Exp Ther.,* 2003, vol. 304 (2), 547-559
- **WALKER, J.M. et al.** *Pharmacological Reviews,* 1990, vol. 42, 355
- **YAKSH, T.L.** Pharmacology of spinal adrenergic systems which modulate spinal nociceptive processing. *Pharmacol Biochem Behav.,* 1985, vol. 22 (5), 845-58